# EUROPEAN PATENT SPECIFICATION

(11) **EP 4 167 961 B1**
(45) Date of publication and mention of the grant of the patent: **29.04.2026**
(21) Application number: 21830096.0
(22) Date of filing: 23.06.2021
(51) Int. Cl.: A61K 9/00, A61K 35/16, A61K 38/42, A61P 7/08

(54) **COMPOSITIONS AND METHODS FOR TREATING HEMORRHAGIC SHOCK**
ZUSAMMENSETZUNGEN UND VERFAHREN ZUR BEHANDLUNG VON HÄMORRHAGISCHEM SCHOCK
COMPOSITIONS ET MÉTHODES DE TRAITEMENT D'UN CHOC HÉMORRAGIQUE

(30) Priority: 23.06.2020 US 202063042668 P
(43) Date of publication of application: 26.04.2023
(73) Proprietor: Virtech Bio, Inc., Natick, MA 01760 (US)
(72) Inventor: LIGHT, William, Richard, III, Natick, MA 01760 (US)
(74) Representative: Carpmaels & Ransford LLP
(86) International application number: PCT/US2021/038606
(87) International publication number: WO 2021/262803

(56) References cited:
- US-A- 4 001 401
- US-A1- 2010 311 657
- US-A1- 2011 256 225
- US-A1- 2020 078 407
- US-B1- 7 135 554
- US-B2- 6 518 010
- MELEDEO MICHAEL ADAM ET AL: "Freeze-dried plasma mitigates the dilution effects of a hemoglobin-based oxygen carrier (HBOC-201) in a model of resuscitation for hemorrhage and hemodilution", vol. 87, no. 1S, 1 July 2019 (2019-07-01), US, pages S83 - S90, XP055781723, ISSN: 2163-0755, Retrieved from the Internet <URL:http://dx.doi.org/10.1097/TA.0000000000002317> DOI: 10.1097/TA.0000000000002317
- BAKER N ET AL: "310 Damage Control Resuscitation: A Trial of Combination of HBOC-201 and Resuscitation Fluids in a Swine Model of Severe Hemorrhagic Shock", ANNALS OF EMERGENCY MEDICINE, vol. 68, no. 4, 1 October 2016 (2016-10-01), XP029781199, ISSN: 0196-0644, DOI: 10.1016/J.ANNEMERGMED.2016.08.326

## Description

### RELATED APPLICATION

This application claims the benefit of United States Provisional Patent Application No. 63/042,668, filed June 23, 2020.

### FIELD OF THE DISCLOSURE

The present disclosure generally relates to the field of hemorrhagic shock, and in particular, to compositions and their use in methods for treating hemorrhagic shock.

### BACKGROUND

The following paragraphs are provided by way of background to the present disclosure.

Hemorrhagic shock is a clinical syndrome that can occur when vital organs cease to function as a result of substantial blood loss from damaged blood vessels. Thus, hemorrhagic shock can occur, for example, following traumatic injury, such as when deep bodily cuts or burns are sustained, or in the course of the performance of surgical procedures, or as a result of any internal bleed, such as a gastrointestinal bleed. Diminished blood flow to vital organs results in an inadequate delivery of oxygen, and when demand of oxygen outweighs supply, the organs, as well as the organism, can enter into a state of shock. Hemorrhagic shock symptoms are generally life threatening and treated as a medical emergency.

The innate bodily process for stopping blood loss from damaged blood vessels is known as hemostasis and involves narrowing of the blood vessels and blood coagulation to form a blood clot which functions as a plug. Well known medical techniques to facilitate hemostasis and treat or prevent hemorrhagic shock, include techniques to stem the flow of blood, including the application of tourniquets and the application of pressure bandages and wound dressings or techniques to intervene in internal bleeding. However, especially in instances of severe blood vessel damage, these techniques often do not adequately succeed in stopping blood loss.

Other known therapies for the treatment of hemorrhagic shock include blood transfusion therapies. These therapies can be very effective, however they critically depend on the availability of appropriately stored blood, and may not be a treatment option in the event of donor blood shortages. In addition, blood transfusion therapy may not be feasible when a patient has sustained an injury at a location distant from a medical care facility, for example, on a battlefield. Furthermore, the blood types of the blood donor and the recipient must be compatible, and the transfused blood must be free of bloodborne pathogenic agents.

Yet other therapies known to the art to treat hemorrhagic shock involve the use of other therapeutic liquids, including blood plasma, and so-called plasma volume expanders, which are intravenously delivered.

In this respect, the administration of blood plasma can increase blood vessel fluid volume to thereby assist the remaining red blood cells to deliver oxygen to vital organs. Furthermore, the administration of blood plasma can aide hemostasis. However, blood plasma preparations do not include an oxygen carrying constituent. Moreover, the shelf life of known blood plasma preparations is relatively short, typically on the order of days, unless the blood plasma preparations are stored frozen.

Plasma volume expanders, crystalloid or colloid based plasma volume expanders, for example, have also been employed to increase blood vessel fluid volume and assist the remaining red blood cells to deliver oxygen to vital organs. It is a drawback of plasma volume expanders, however, that the administration thereof can result in an undesirable dilution of red blood cells, and a delay in hemostasis, and can contribute to trauma induced or other type of coagulopathy, *i.e.* an impairment of blood coagulation. Furthermore, the absence of an oxygen carrying constituent within preparations of plasma volume expanders limits the utility of these preparations in the treatment of hemorrhagic shock.

To overcome several of the foregoing deficiencies, a class of compounds based on hemoglobin, the natural oxygen carrying protein constituent present in red blood cells, has evolved. Hemoglobin-based oxygen carriers, also commonly referred to as HBOCs, are deemed desirable since they allow both for blood vessel fluid volume expansion and can deliver oxygen. Furthermore, some HBOC preparations do not require refrigeration and do not carry blood borne disease contaminants. However, one significant drawback associated with known HBOC preparations is that the administration thereof can cause high blood pressure and delayed hemostasis due to hemodilution of coagulation plasma components. These features render known HBOC preparations insufficiently suitable for the treatment of hemorrhagic shock. Indeed, in the United States the Food and Drug Administration (FDA) has currently not yet approved HBOC based therapies to treat hemorrhagic shock (Gupta, A.S., 2019, Shock 52(1S): 70-83).

Thus, it will be clear that although medical techniques for treating hemorrhagic shock are known to the art, many drawbacks associated with known techniques remain. There is therefore a need in the art for improved techniques for treating hemorrhagic shock, and in particular, there is a need for improved fluid-based therapies for the treatment of hemorrhagic shock.

### SUMMARY

The invention is defined in the claims. The following paragraphs are intended to introduce the reader to the more detailed description that follows and not to define or limit the claimed subject matter of the present disclosure.

In one broad aspect, the present disclosure generally relates to hemorrhagic shock, and to pharmaceutical formulations for the prevention or treatment of hemorrhagic shock.

According to one aspect, in accordance with the teachings herein, the present disclosure provides, in at least one embodiment, a liquid pharmaceutical formulation comprising an oxygenation complement comprising a hemoglobin-based oxygen carrier (HBOC) and blood plasma.

In at least one embodiment, in an aspect, the oxygenation complement can comprise an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent.

In at least one embodiment, in an aspect, the pharmaceutical formulation can comprise:
up to about 95% by weight of the oxygenation complement, the oxygenation complement comprising an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and
the balance by weight of the pharmaceutical formulation being constituted by a pharmaceutically acceptable carrier, diluent, or auxiliary agent.

In at least one embodiment, in an aspect, the oxygenation complement can comprise an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent.

In at least one embodiment, in an aspect, the pharmaceutical formulation can comprise:
up to about 95% by weight of the oxygenation complement, the oxygenation complement comprising an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and
the balance by weight of the pharmaceutical formulation being constituted by a pharmaceutically acceptable carrier, diluent, or auxiliary agent.

In at least one embodiment, in an aspect, the HBOC can be a hyperpolymerized HBOC.

In at least one embodiment, in an aspect, the auxiliary agent can comprise blood platelets.

In another aspect, the present disclosure provides, in at least one embodiment, a method of preparing a liquid pharmaceutical formulation for the prevention or treatment of hemorrhagic shock, the method comprising: mixing
(i) a hemoglobin-based oxygen carrier (HBOC) preparation; and
(ii) a blood plasma preparation;
to thereby constitute an oxygenation complement or formulate a liquid pharmaceutical formulation.

In at least one embodiment, in an aspect, at least one of the HBOC preparation and the blood plasma preparation can be a liquid preparation.

In at least one embodiment, in an aspect, at least one of the blood plasma preparation and the HBOC preparation can be a dried blood plasma preparation or a dried HBOC preparation, respectively, having a moisture content of from about 1% (w/w) to about 10% (w/w).

In at least one embodiment, in an aspect, the blood plasma preparation can be a dried blood plasma preparation having a moisture content from about 1% (w/w) to about 10% (w/w), and the HBOC preparation can be a liquid low ionic solute HBOC preparation.

In at least one embodiment, in an aspect, the liquid HBOC preparation can be a low ionic solute HBOC preparation comprising no more than about 13.5 mEq/L sodium ions (Na⁺), no more than about 0.35 mEq/L potassium ions (K⁺) ions, no more than about 0.46 mEq/L calcium ions (Ca²⁺), no more than about 0.15 mEq/L magnesium ions (Mg²⁺) ions, no more than about 9.6 mEq/L chloride ions (CI-) ions, no more than 2.4 mEq/L bicarbonate ions (HCO₃²⁻), and no more than about 0.25 mEq/L hydrogen phosphate ions (HPO₄²⁻).

In at least embodiment, in an aspect, the method can further comprise mixing (iii) a pharmaceutically acceptable carrier, diluent, or auxiliary agent together with the oxygenation complement to thereby form a liquid pharmaceutical formulation.

In at least one embodiment, in an aspect, the oxygenation complement can be constituted to comprise an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent.

In at least one embodiment, in an aspect, the liquid formulation can be formulated by formulating therein up to 95% by weight of the oxygenation complement, the oxygenation complement comprising an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and the balance by weight of the pharmaceutical formulation being constituted by the pharmaceutically acceptable carrier, diluent, or auxiliary agent.

In at least one embodiment, in an aspect, the oxygenation complement can be constituted to comprise an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent.

In at least one embodiment, in an aspect, the liquid formulation can be formulated by formulating therein up to 95% by weight of the oxygenation complement, the oxygenation complement comprising an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and the balance by weight of the pharmaceutical formulation being constituted by the pharmaceutically acceptable carrier, diluent, or auxiliary agent.

In at least one embodiment, the auxiliary agent can comprise blood platelets.

In at least one embodiment, in an aspect, the HBOC preparation and the blood plasma preparation can be mixed 1 hour or less prior to administration to a subject in need thereof.

In another aspect, the present disclosure provides, in at least one embodiment, a method for preventing or treating hemorrhagic shock, the method comprising intravenously administering to a subject in need thereof an effective amount of an oxygenation complement comprising a hemoglobin-based oxygen carrier (HBOC) and blood plasma, in an effective amount to prevent or treat hemorrhagic shock in the subject.

In at least one embodiment, in an aspect, the oxygenation complement can be administered to the subject by consecutive administration of a liquid HBOC formulation and a liquid blood plasma formulation, wherein the liquid HBOC formulation is substantially free of blood plasma, and wherein the liquid blood plasma formulation is substantially free of HBOC.

In at least one embodiment, in an aspect, the oxygenation complement can be administered to the subject by consecutive administration of the HBOC formulation and the blood plasma formulation, wherein the HBOC formulation is administered prior to the blood plasma formulation.

In at least one embodiment, in an aspect, the oxygenation complement can be administered to the subject by consecutive delivery of the HBOC formulation and the blood plasma formulation, wherein the blood plasma formulation administered prior to the HBOC formulation.

In at least one embodiment, in an aspect, the HBOC formulation and the blood plasma formulation can be administered to the subject no more than about 6 hours apart from each other.

In at least one embodiment, in an aspect, the oxygenation complement can be administered by administering a liquid pharmaceutical formulation having been prepared by mixing an HBOC preparation and a blood plasma preparation.

In at least one embodiment, in aspect, the liquid pharmaceutical formulation can further comprise a pharmaceutically acceptable carrier, diluent or auxiliary agent.

In at least one embodiment, in an aspect, the oxygenation complement can comprise an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent.

In at least one embodiment, in an aspect, the liquid pharmaceutical formulation can comprise
up to 95% by weight of the oxygenation complement, the oxygenation complement comprising an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and
the balance by weight of the pharmaceutical formulation being constituted by the pharmaceutically acceptable carrier, diluent, or auxiliary agent.

In at least one embodiment, in an aspect, the oxygenation complement can comprise an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent.

In at least one embodiment, in an aspect, the liquid pharmaceutical formulation can comprise
up to 95% by weight of the oxygenation complement, the oxygenation complement comprising an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and
the balance by weight of the pharmaceutical formulation being constituted by the pharmaceutically acceptable carrier, diluent, or auxiliary agent.

In at least one embodiment, in an aspect, the auxiliary agent can comprise blood platelets.

In at least one embodiment, in aspect, a pharmaceutical formulation comprising blood platelets can be co-administered with the oxygenation complement.

In at least one embodiment, in an aspect, the hemorrhagic shock can be caused by the subject having experienced traumatic blood loss.

In at least one embodiment, in an aspect, the hemorrhagic shock can be caused by the subject by having experienced non-traumatic blood loss.

In another aspect, the present disclosure provides, in at least one embodiment, a pharmaceutical package comprising (i) a first package comprising a hemoglobin-based oxygen carrier (HBOC) preparation and (ii) a second package comprising a blood plasma preparation, the pharmaceutical package capable of being used to constitute a liquid pharmaceutical formulation by mixing of the HBOC preparation and the blood plasma preparation, the formed liquid pharmaceutical formulation capable of being used to prevent or treat hemorrhagic shock when administered to a subject in need thereof, the pharmaceutical package further including instructions to prepare the liquid pharmaceutical formulation.

In at least one embodiment, in an aspect, the pharmaceutical package can further comprise (iii) a third package comprising a pharmaceutically acceptable carrier, diluent or auxiliary agent for mixing with the HBOC preparation and the blood plasma preparation to thereby form the liquid pharmaceutical formulation.

In at least one embodiment, in an aspect, at least one of the HBOC preparation and the blood plasma preparation can be a dried HBOC preparation or blood plasma preparation, having a moisture content from about 1% (w/w) to about 10% (w/w).

In at least one embodiment, in an aspect, the blood plasma preparation can be a dried HBOC preparation having a moisture content from about 1% (w/w) to about 10% (w/w), and the HBOC preparation can be a low ionic solute HBOC preparation.

In at least one embodiment, in an aspect, the low ionic solute HBOC preparation can comprise no more than about 13.5 mEq/L sodium ions (Na⁺), no more than about 0.35 mEq/L potassium ions (K⁺) ions, no more than about 0.46 mEq/L calcium ions (Ca²⁺), no more than about 0.15 mEq/L magnesium ions (Mg²⁺) ions, no more than about 9.6 mEq/L chloride ions (CI-) ions, no more than 2.4 mEq/L bicarbonate ions (HCO₃²⁻), and no more than about 0.25 mEq/L hydrogen phosphate ions (HPO₄²⁻).

In another aspect, the present disclosure provides, in at least one embodiment a liquid low ionic solute HBOC preparation comprising no more than about than about 27 milliequivalent (mEq/L) sodium ions (Na⁺), no more than about 0.7 mEq/L potassium ions (K⁺) ions, no more than about 0.92 mEq/L calcium ions (Ca²⁺), no more than about 0.3 mEq/L magnesium ions (Mg²⁺) ions, no more than about 19.2 mEq/L chloride ions (CI-) ions, no more than 4.8 mEq/L bicarbonate ions (HCO₃²⁻), and no more than about 0.5 mEq/L hydrogen phosphate ions (HPO₄²⁻).

In at least one embodiment, in an aspect, the liquid low ionic solute HBOC preparation can comprise no more than about 13.5 mEq/L sodium ions (Na⁺), no more than about 0.35 mEq/L potassium ions (K⁺) ions, no more than about 0.46 mEq/L calcium ions (Ca²⁺), no more than about 0.15 mEq/L magnesium ions (Mg²⁺) ions, no more than about 9.6 mEq/L chloride ions (CI-) ions, no more than 2.4 mEq/L bicarbonate ions (HCO₃²⁻), and no more than about 0.25 mEq/L hydrogen phosphate ions (HPO₄²⁻).

In at least one embodiment, in an aspect, the liquid low ionic solute HBOC preparation can comprise no more than about 6.75 mEq/L sodium ions (Na⁺), no more than about 0.175 mEq/L potassium ions (K⁺) ions, no more than about 0.23 mEq/L calcium ions (Ca²⁺), no more than about 0.075 mEq/L magnesium ions (Mg²⁺) ions, no more than about 4.8 mEq/L chloride ions (CI-) ions, no more than 1.2 mEq/L bicarbonate ions (HCO₃²⁻), and no more than about 0.125 mEq/L hydrogen phosphate ions (HPO₄²⁻).

In at least one embodiment, in an aspect, the low ionic solute HBOC preparation can comprise an HBOC quantity of from about 5% to about 50% by weight, the balance of the preparation comprising a pharmaceutically acceptable diluent.

In another aspect, the present disclosure provides, in at least one embodiment, a use of a hemoglobin-based oxygen carrier (HBOC) and blood plasma to prepare an oxygenation complement to formulate a liquid pharmaceutical formulation for the prevention or treatment of hemorrhagic shock.

In another aspect, the present disclosure provides, in at least one embodiment, a use of a liquid pharmaceutical formulation to prevent or treat hemorrhagic shock, the liquid formulation comprising an oxygenation complement comprising a hemoglobin-based oxygen carrier (HBOC) and blood plasma and a pharmaceutically acceptable carrier, diluent or auxiliary agent.

In another aspect, the present disclosure provides, in at least one embodiment, a liquid pharmaceutical formulation for use in preventing or treating hemorrhagic shock, the formulation comprising an oxygenation complement comprising a hemoglobin-based oxygen carrier (HBOC) and blood plasma and a pharmaceutically acceptable carrier, diluent or auxiliary agent.

In another aspect, the present disclosure provides, in at least one embodiment, a use of a liquid pharmaceutical formulation in the manufacture of a medicament for use in preventing or treating hemorrhagic shock, the formulation comprising an oxygenation complement comprising a hemoglobin-based oxygen carrier (HBOC) and blood plasma or blood plasma constituents and a pharmaceutically acceptable carrier, diluent or auxiliary agent.

Other features and advantages or the present disclosure will become apparent from the following detailed description. It should be understood, however, that the detailed description, while indicating preferred embodiments or implementations of the present disclosure, is given by way of illustration only, since various changes and modification within the spirit and scope of the disclosure will become apparent to those of skill in the art from the detailed description.

### BRIEF DESCRIPTION OF THE DRAWINGS

These and other features of the disclosure will become more apparent in the following detailed description in which reference is made to the appended drawings. The figures are provided herein for a better understanding of the example embodiments and to show more clearly how the various example embodiments may be carried into effect. The figures are not intended to limit the present disclosure.

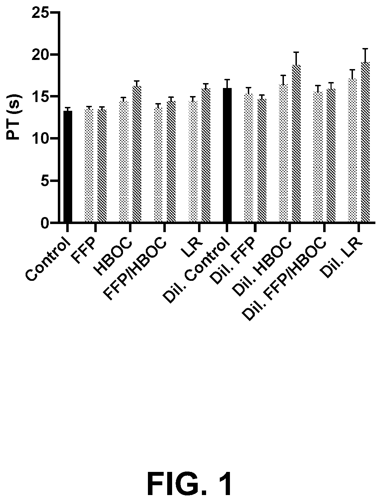

**FIG. 1** is a bar graph representing results if the performance of certain experiments described herein. Shown is prothrombin time (PT) (PT (s)) for various tested assay mixtures comprising whole blood (Control), fresh frozen plasma (FFP), fresh frozen plasma + HBOC (FFP + HBOC), Lactated-Ringer's solution (LR), whole blood + PlasmaLyte (Dil. Control), fresh frozen plasma + PlasmaLyte (Dil. FFP), fresh frozen plasma + HBOC + PlasmaLyte (Dil. FFP + HBOC), Lactated-Ringer's solution + PlasmaLyte (Dil. LR); light grey bars represent 10% dilutions, dark grey bars represent 25% dilutions; standard deviation is n = 6.

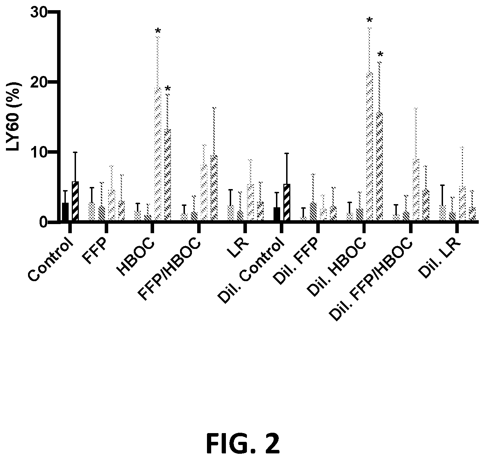

**FIG. 2** is a bar graph representing results if the performance of certain experiments described herein. Shown is clot lysis (LY60(%)) for various tested assay mixtures (with and without tPA) comprising whole blood (Control), fresh frozen plasma (FFP), fresh frozen plasma + HBOC (FFP + HBOC), Lactated-Ringer's solution (LR), whole blood + PlasmaLyte (Dil. Control), fresh frozen plasma + PlasmaLyte (Dil. FFP), fresh frozen plasma + HBOC + PlasmaLyte (Dil. FFP + HBOC), Lactated-Ringer's solution + PlasmaLyte (Dil. LR); light grey bars represent 10% dilutions, dark grey bars represent 25% dilutions; light striped grey bars represent 10% dilutions with tPA, dark striped grey bars represent 20% dilutions with tPA; standard deviation is n = 6.

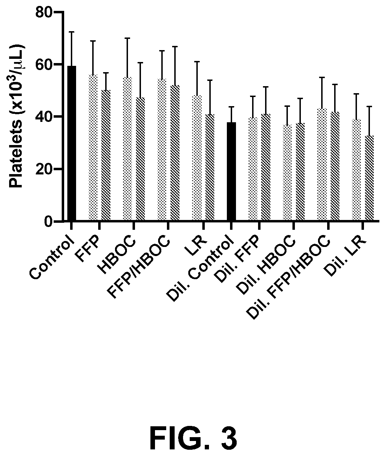

**FIG. 3** is a bar graph representing results if the performance of certain experiments described herein. Shown is platelet count (platelets x10³/µl)) for various tested assay mixtures comprising whole blood (Control), fresh frozen plasma (FFP), fresh frozen plasma + HBOC (FFP + HBOC), Lactated-Ringer's solution (LR), whole blood + PlasmaLyte (Dil. Control), fresh frozen plasma + PlasmaLyte (Dil. FFP), fresh frozen plasma + HBOC + PlasmaLyte (Dil. FFP + HBOC), Lactated-Ringer's solution + PlasmaLyte (Dil. LR); light grey bars represent 10% dilutions, dark grey bars represent 25% dilutions; standard deviation is n = 6.

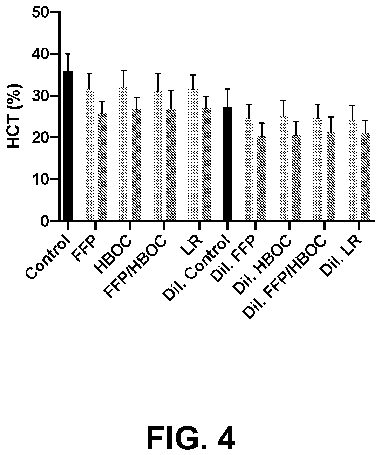

**FIG. 4** is a bar graph representing results if the performance of certain experiments described herein. Shown is hematocrit content (HCT (%)) for various tested assay mixtures comprising whole blood (Control), fresh frozen plasma (FFP), fresh frozen plasma + HBOC (FFP + HBOC), Lactated-Ringer's solution (LR), whole blood + PlasmaLyte (Dil. Control), fresh frozen plasma + PlasmaLyte (Dil. FFP), fresh frozen plasma + HBOC + PlasmaLyte (Dil. FFP + HBOC), Lactated-Ringer's solution + PlasmaLyte (Dil. LR); light grey bars represent 10% dilutions, dark grey bars represent 25% dilutions; standard deviation is n = 6.

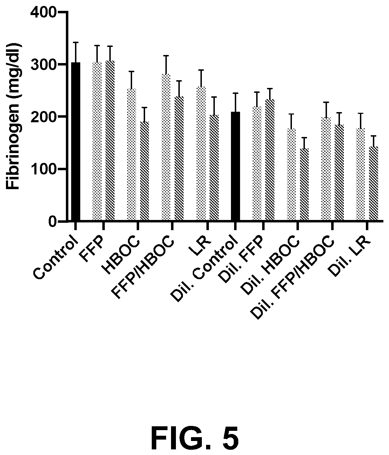

**FIG. 5** is a bar graph representing results if the performance of certain experiments described herein. Shown is fibrinogen concentration ( fibrinogen mg/dl) for various tested assay mixtures comprising whole blood (Control), fresh frozen plasma (FFP), fresh frozen plasma + HBOC (FFP + HBOC), Lactated-Ringer's solution (LR), whole blood + PlasmaLyte (Dil. Control), fresh frozen plasma + PlasmaLyte (Dil. FFP), fresh frozen plasma + HBOC + PlasmaLyte (Dil. FFP + HBOC), Lactated-Ringer's solution + PlasmaLyte (Dil. LR); light grey bars represent 10% dilutions, dark grey bars represent 25% dilutions; standard deviation is n = 6.

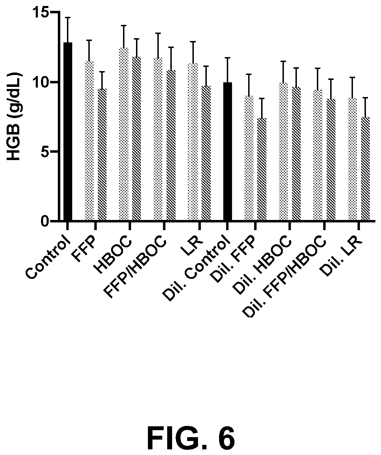

**FIG. 6** is a bar graph representing results if the performance of certain experiments described herein. Shown is hemoglobin concentration (HGB g/dl) for various tested assay mixtures comprising whole blood (Control), fresh frozen plasma (FFP), fresh frozen plasma + HBOC (FFP + HBOC), Lactated-Ringer's solution (LR), whole blood + PlasmaLyte (Dil. Control), fresh frozen plasma + PlasmaLyte (Dil. FFP), fresh frozen plasma + HBOC + PlasmaLyte (Dil. FFP + HBOC), Lactated-Ringer's solution + PlasmaLyte (Dil. LR); light grey bars represent 10% dilutions, dark grey bars represent 25% dilutions; standard deviation is n = 6.

The figures together with the following detailed description makes apparent to those of skill in the art how the disclosure may be implemented in practice.

### DETAILED DESCRIPTION

Various processes, methods and compositions will be described below to provide an example of an embodiment of the claimed subject matter. No embodiment described below limits any claimed subject matter and any claimed subject matter may cover processes, methods, or compositions that differ from those described below. The claimed subject matter is not limited to any process, method, or composition having all of the features of processes, methods, or compositions described below, or to features common to multiple or processes, methods, compositions or compositions described below. It is possible that a process, method, or composition described below is not an embodiment of any claimed subject matter. Any subject matter disclosed in processes, methods, or compositions described below that is not claimed in this document may be the subject matter of another protective instrument, for example, a continuing patent application, and the applicants, inventors or owners do not intend to abandon, disclaim or dedicate to the public any such subject matter by its disclosure in this document.

As used herein and in the claims, the singular forms, such "a", "an" and "the" include the plural reference and vice versa unless the context clearly indicates otherwise. Throughout this specification, unless otherwise indicated, "comprise," "comprises" and "comprising" are used inclusively rather than exclusively, so that a stated integer or group of integers may include one or more other non-stated integers or groups of integers. The term "or" is inclusive unless modified, for example, by "either". The term "and/or" is intended to represent an inclusive or. That is "X and/or Y" is intended to mean X or Y or both X and Y, for example. As a further example, X, Y and/or Z is intended to mean X or Y or Z or any combination thereof

When ranges are used herein for physical properties, such as molecular weight, or chemical properties, such as chemical formulae, all combinations and sub-combinations of ranges and specific embodiments therein are intended to be included. Other than in the operating examples, or where otherwise indicated, all numbers expressing quantities of ingredients or reaction conditions used herein should be understood as modified in all instances by the term "about." The term "about" when referring to a number or a numerical range means that the number or numerical range referred to is an approximation within experimental variability (or within statistical experimental error), and thus the number or numerical range may vary between 1% and 15% of the stated number or numerical range, as will be readily recognized by context. Furthermore, any range of values described herein is intended to specifically include the limiting values of the range, and any intermediate value or sub-range within the given range, and all such intermediate values and sub-ranges are individually and specifically disclosed (e.g. a range of 1 to 5 includes 1, 1.5, 2, 2.75, 3, 3.90, 4, and 5). Similarly, other terms of degree such as "substantially" and "approximately" as used herein mean a reasonable amount of deviation of the modified term such that the end result is not significantly changed. These terms of degree should be construed as including a deviation of the modified term if this deviation would not negate the meaning of the term it modifies.

Unless otherwise defined, scientific and technical terms used in connection with the formulations described herein shall have the meanings that are commonly understood by those of ordinary skill in the art. The terminology used herein is for the purpose of describing particular embodiments, only, and is not intended to limit the scope of the subject matter of the present disclosure, which is defined solely by the claims.

### Definitions

The term "effective amount", as used herein, refers to an amount of an active agent or pharmaceutical formulation sufficient to induce a desired biological or therapeutic effect. Such effect can include an effect with respect to the signs, symptoms or causes of an injury, disorder, or disease or any other desired alteration of a biological system. The effective amount can vary depending, for example, on the health condition, injury stage, disorder stage, or disease stage of a subject being treated, timing of the administration, manner of the administration, age of the subject, and the like, all of which can be determined by those of skill in the art.

The terms "HBOC", or "hemoglobin-based oxygen carrier", as may be used interchangeably herein, refer to an oxygen carrying hemoglobin derivative wherein the hemoglobin molecule has been modified, for example, by chemically cross-linking of hemoglobin molecules, polymerization, or by conjugation of hemoglobin to other molecules, for example, conjugation to polyethylene glycol (PEG) or dextran.

The term "hemoglobin", as used herein, refers to the protein contained within red blood cells that transports oxygen in living organisms. Each molecule of hemoglobin includes 4 subunits, 2 α-chains and 2 β-chains, which together are configured in a tetrameric structure. Each subunit also contains an iron containing heme group, which binds oxygen. Thus, each hemoglobin molecule can bind 4 oxygen molecules. As used herein, the term by itself refers to native hemoglobin, including naturally occurring variants thereof, and further includes hemoglobin obtainable from any invertebrate or vertebrate animal, or a human, including, without limitation, e.g. human hemoglobin, bovine hemoglobin, ovine hemoglobin, and porcine hemoglobin, and any recombinant hemoglobin.

The term "hemorrhagic shock", as used herein, refers to a condition in a subject induced by the loss of a substantial quantity of the subject's total blood volume, and resulting into an inadequate flow of blood through vital organs, including the heart and the brain, such that normal physiological organ functioning is compromised. The loss of blood can be caused by trauma, including blunt or penetrating trauma, such as an injury, a deep cut or a burn, for example, or trauma sustained in the performance of a surgical operation. Substantial blood loss can also occur as a result of non-traumatic bleeding, for example, gastrointestinal bleeding, such as may be caused by, for example, a peptic ulcer or any other gastrointestinal disorder. Substantial blood loss includes the loss of at least 15%, or about 15%, at least 20%, or about 20%, at least 30%, or about 30%, at least 40% or about 40% of the total blood volume of a subject.

The term "hemostasis", as used herein, refers to the cessation of blood loss from one or more damaged blood vessels, including arteries, veins or capillaries, as well as diffuse blood loss from tissue or an organ. Hemostasis includes a constriction of blood vessels and blood coagulation to form a blood clot, functioning as a plug.

The term "oxygenation complement" refers to blood plasma and HBOC together. The oxygenation complement can be constituted as a liquid mixture comprising blood plasma and HBOC. The oxygenation complement can aide and complement existing oxygenation constituents in a subject's circulatory system upon administration thereof to the subject. The term also can also refer to the more or less contemporaneous administration of a separate blood plasma formulation and HBOC formulation to a subject.

The term "pharmaceutical formulation", as used herein, refers to an active agent, which together with at least one of a carrier, diluent, or auxiliary agent is suitable for therapeutic administration. In the context of the present disclosure, the active agent can be an oxygenation complement comprising HBOC and blood plasma. A pharmaceutical formulation can also be, in certain embodiments, hereof a blood plasma formulation or an HBOC formulation suitable for therapeutic administration.

The term "pharmaceutically acceptable", as used herein, refers to materials, including carriers, diluents, or auxiliary agent that are compatible with other materials in a pharmaceutical formulation and within the scope of reasonable medical judgement suitable for use in contact with human beings or animals without excessive toxicity, allergic response, irritation, or other adverse response commensurate with a reasonable risk/benefit ratio.

The term "preparation", as used herein, refers to a composition which is suitable for use in the manufacture of a pharmaceutical formulation. Thus, for example, an HBOC preparation refers to a composition comprising HBOC suitable for use in the manufacture of a pharmaceutical formulation, a pharmaceutical formulation for the treatment of hemorrhagic shock, for example; and a blood plasma preparation refers to a composition comprising blood plasma suitable for use in the manufacture of a pharmaceutical formulation, a pharmaceutical formulation for the treatment of hemorrhagic shock, for example. Manufacturing of a pharmaceutical formulation may involve, for example, formulation with a pharmaceutically acceptable diluent or carrier or auxiliary agent.

The term "red blood cell" or "erythrocyte", as used herein, means a non-nucleated cell containing hemoglobin that circulates in blood, generally responsible for the red color of blood.

The term "subject", as used herein, refers to an animal, a mammal, or yet further, a human.

The terms "treating" and "treatment", and the like, as used herein, are intended to mean obtaining a desirable physiological, pharmacological, or biological effect. The effect may result in amelioration of a sign, symptom or cause of an injury, disorder, or disease, attributable to the injury, disorder, or disease, or the effect may result in partially or completely curing or healing of an injury, disorder, or disease. Clinical evidence of treatment of may vary with the injury, disorder, or disease, the subject and the selected treatment. In the context of the treatment of hemorrhagic shock, a physiological effect may include hemostasis.

"Substantially pure", as used herein, herein describes an entity, e.g. a cell or chemical or biochemical compound, such as hemoglobin, which has been separated from constituents that naturally accompany it. Typically, an entity is substantially pure when at least 60%, more preferably at least 75%, more preferably at least 90%, 95%, 96%, 97%, or 98%, and most preferably at least 99% of the total material (by volume, by wet or dry weight, or by mole percent or mole fraction) in a sample is the entity of interest. With reference to a red blood cell preparation, the term can further refer to the separation from of non-red blood cells, *e.g.* white blood cells and thrombocytes, including a preparation comprising no more than 2%, 1%, 0.5%, or 0.1% by weight of non-red blood cells. Furthermore, with respect to blood plasma, the term can further refer to the separation from blood cells, e.g. red blood cells, white blood and thrombocytes, including a preparation comprising no more than 5%, 4%, 3%, 2% or 1% by weight of blood cells. Purity can be measured by any appropriate method, *e.g.,* in the case of proteins, by chromatography, gel electrophoresis or HPLC analysis, and in the case of red blood cells, flow cytometry.

### General Implementation

In overview, it has been realized, that novel pharmaceutical formulations comprising an oxygenation complement including a hemoglobin-based oxygen carrier (HBOC) and blood plasma component(s) can be prepared and, that these pharmaceutical formulations can be unexpectedly and effectively used to prevent or treat hemorrhagic shock. The pharmaceutical formulations of the present disclosure can be administered to a subject in need thereof, notably, a subject at risk of suffering a hemorrhagic shock, or a subject suffering a hemorrhagic shock, for example, a hemorrhagic shock resulting from a traumatic injury. The pharmaceutical formulations of the present disclosure can complement the existing oxygenation constituents in a subject's circulatory system upon administration thereof to a subject. Surprisingly, upon administration of the pharmaceutical formulations of the present disclosure, the blood loss from injured blood vessels can be brought under control, and hemostasis can be rapidly attained. Thus, the pharmaceutical formulations of the present disclosure can be used to prevent or treat hemorrhagic shock. In this respect, it is noted that the efficacy of the administration of a formulation comprising HBOC alone or blood plasma alone, as an active compound, can be said to be substantially inferior to the efficacy of the administration of the pharmaceutical formulations of the present disclosure. In particular, the administration of preparations comprising HBOC alone as an active agent may lead to an increase in blood pressure and a delay in the time needed to attain hemostasis, rendering preparations containing HBOC alone as an active agent insufficiently suitable to prevent or treat hemorrhagic shock. The pharmaceutical formulations of the present disclosure can be safely and economically manufactured and methods for manufacture are also included herein.

Furthermore, in certain embodiments hereof, the pharmaceutical formulations of the present disclosure may be constituted in a manner that results in light-weight pharmaceutical products. This renders the pharmaceutical formulations of the present disclosure suitable particularly suitable for use in, for example, battlefield operations, where the use of compact, light-weight medical supplies can be advantageous.

Thus, the disclosure provides, in an aspect, pharmaceutical formulations for the prevention and treatment of hemorrhagic shock. The disclosure provides, in a further aspect, methods for preparing pharmaceutical formulations for the prevention and treatment of hemorrhagic shock. Furthermore, the present disclosure, in yet another aspect, methods for preventing and treatment of hemorrhagic shock.

In what follows selected embodiments are described.

Accordingly, the present disclosure provides, in an aspect, in one embodiment, a pharmaceutical formulation comprising an oxygenation complement comprising a hemoglobin-based oxygen carrier (HBOC) and blood plasma. The pharmaceutical formulation can further comprise a pharmaceutically acceptable carrier, diluent or auxiliary agent. The pharmaceutical formulation can be used to treat or prevent hemorrhagic shock.

In general, the pharmaceutical formulations of the present disclosure can be prepared by initially providing an HBOC preparation and a blood plasma preparation. Thus, next initially example methods to obtain plasma and HBOC formulations will be discussed. Thereafter, methods for preparing pharmaceutical formulations comprising both of these preparations will be discussed. Thereafter methods for preventing or treating hemorrhagic shock will be discussed.

In an aspect, both HBOC preparations and plasma preparations can be manufactured using whole blood as a source. It is noted that blood includes all components of blood circulating in a subject including, but not limited to, red blood cells, white blood cells, plasma, clotting factors, small proteins, platelets and/or cryoprecipitate. This is typically the type of blood which is donated when a human patient gives blood. Suitable blood that may be used in accordance herewith includes invertebrate or vertebrate animal or human blood, including, without limitation, mammalian and avian blood, including, without limitation, human blood, bovine blood, porcine blood, equine blood, ovine blood, or any recombinant hemoglobin formulations. Blood solutions may be collected from live or dead organisms, and may be collected using any techniques and devices known to the art, including, for example, the methodologies described in U.S. Patents 5,084,558 and 5,296,465. The blood may be fresh or from an older sample, for example, blood stored at a blood bank institute. It is preferred that the blood solution is screened for the presence of blood borne pathogens, for example, where human blood is used, HIV and hepatitis B. Preferably, blood free of blood borne pathogens is selected for use in accordance with the methods of the present disclosure. Upon blood collection, it is preferred that an anti-coagulant is added to the blood to prevent blood clotting. Anti-coagulants that may be used include heparin, hirudin, sodium citrate, or ethylenediaminetetraacetic acid (EDTA), for example. The anticoagulant may be provided as an aqueous solution or in particulate form. The anti-coagulant furthermore may be included in a collection vessel prior to collection of blood in the collection vessel, for example, blood may be collected in a heparin coated blood collection tube.

Blood plasma is known in the art as the yellow liquid component of blood, in which the blood cells in whole blood are suspended. It makes up about 55% of the total blood volume and has a density of approximately 1.025 kg/l. Blood plasma includes water (up to 95% by volume), suspended blood plasma proteins, including serum albumins, globulins and fibrinogen, glucose, clotting factors, and electrolytes (*e.g*. Na⁺, Ca²⁺, Mg²⁺, HCO₃⁻, Cl⁻). One well known technique for preparing a blood plasma fraction from whole blood is based on the centrifugation of whole blood samples. Thus, in an aspect, blood samples can be collected in a centrifugation vessel, such as a centrifugation tube, and centrifuged at high speed, for example, for about 10 minutes to about 20 minutes at about 1,000 - 2,000x g using a refrigerated centrifuge. The blood cells will accumulate at the bottom of the centrifugation vessel and blood plasma may collected by be pouring or drawing off the supernatant. The collected blood plasma fraction can be said to be an undiluted plasma preparation, *i.e.* a plasma preparation containing substantially nothing else than all or approximately all of the blood plasma constituents natively present in blood, and in approximately the same relative quantities as those natively present in blood. Blood plasma samples are generally preferably maintained at about 2 ºC to about 8 ºC for immediate use or can be stored for longer periods of time at temperatures of -20 ºC or lower. Other suitable methods for isolating blood plasma from blood cells that may also be used include filtration-based methods, including the methods described in U.S. Patent Nos 10,088,469; 6,403,384 and 5,876,605, for example. By selecting and performing these or other techniques, combinations, or adjustments thereof, a blood plasma preparation may be obtained and used in accordance with the present disclosure. Depending on the selected technique or techniques, the purity of the blood plasma preparation may vary. In preferred embodiments, the blood plasma preparation is a preparation which is substantially free of red blood cells, and contains, for example, less than about 0.5% or less than about 0.1% by weight red blood cells.

Thus, in some embodiments, the blood plasma preparation can be an undiluted liquid preparation.

In an aspect, an undiluted liquid blood plasma preparation may be diluted using a pharmaceutically acceptable diluent, for example, a saline solution. Thus, for example, a weight of an undiluted liquid blood plasma preparation may be diluted with from about 0.5x to about 10x weights of a diluent, to form a diluted liquid blood plasma preparation. For the purposes of the present disclosure, a diluted liquid blood plasma preparation can be said to contain a certain weight percentage of undiluted liquid blood plasma preparation. Thus, for example, when a weight of an undiluted liquid blood plasma preparation is diluted with 9 weights of a diluent, the obtained diluted liquid plasma preparation can be said to contain 10% by weight of undiluted liquid plasma preparation. Furthermore, when a dried blood plasma preparation is prepared, as hereinafter further described, the dried blood plasma preparation may be a preparation from which, for example, 90% by weight of the endogenous liquid may have been removed, and thus the dried blood plasma preparation can be said to contain about 10% by weight of undiluted liquid plasma preparation.

In some embodiments, in an aspect, the blood plasma preparation can be a dried blood plasma preparation. In general, in order to obtain a dried blood plasma preparation, a blood plasma preparation in a liquid state is treated to transform the liquid blood plasma preparation into a particulate blood plasma preparation. A dried blood plasma preparation in accordance herewith can be a blood plasma preparation having a moisture content of about 10% (w/w) or less, for example, from about 1% (w/w) to about 10% (w/w), or from about 1% (w/w) to about 8% (w/w), or from about 1% (w/w) to about 5% (w/w), or from about 1% (w/w) to about 3% (w/w), or from about 1% (w/w) to about 2% (w/w). In this respect, a dried blood plasma preparation can be prepared, for example, by spray drying or freeze drying (also known to those of skill in the art as lyophilizing) a liquid blood plasma preparation.

As will be known by those of skill in the art, in general, freeze drying involves freezing of a liquid suspension, and drying by sublimation, typically under partial vacuum conditions, and subsequent separation of the dried product and elimination of sublimated moisture. Techniques for freeze drying blood plasma are known to the art and may be selected and used by those of skill in the art to obtain a dried blood plasma preparation. These techniques include, for example, the techniques for freeze drying blood plasma described in U.S. Patents 7,931,919 and 8,518,452, both of which are incorporated herein by reference.

Spray drying, as will be known to those of skill in the art, in general, involves spraying of a liquid suspension, using, for example, a pressure nozzle or atomizer into a warm drying medium, typically air, although other gases such as nitrogen can also be used, in a controlled fashion. Upon contact between the sprayed liquid droplets and the warm drying medium droplet moisture evaporates, and a product with a desired moisture content can be attained. The product is then separated from the drying medium. Techniques for spray drying blood plasma which in this respect may be used include the tecliniques for spray drying blood plasma described in U.S. Patent 9,545,397 and U.S. Patent Application having Publication No US2018/1015381 1, both of which are incorporated herein by reference.

Dried blood plasma may also be commercially purchased, for example, from Velico Medical^{®}, Inc. (Beverly, MA.) or Teleflex^{®}, Inc. (Wayne, PA.).

Turning next to HBOC preparations and methods of making HBOC preparations, in one embodiment, isolated red blood cells may be used as a source from which hemoglobin can be extracted to obtain an isolated hemoglobin preparation. The hemoglobin preparation in turn can be used to prepare an HBOC preparation.

Red blood cells constitute about 45% of the total blood volume. Techniques that may be used to separate red blood cells from whole blood includes centrifugation and/or straining and filtering techniques, or a combination thereof. Centrifugation techniques can involve the collection of blood samples in a centrifugation vessel, such as a centrifugation tube, and centrifugation for example, for about 10 to about 20 minutes at about 500 - 1,000 x g using a refrigerated centrifuge. The red blood cells will accumulate as a pellet at the bottom of the centrifugation vessel and can be collected by pouring off the blood plasma supernatant. The red blood cells can then be resuspended in a suitable isotonic buffer, for example, 0.9% NaCl, 5mM sodium phosphate, pH 8. Centrifugation and resuspension may be repeated several times to wash the red blood cells and remove soluble impurities. It may also be desirable to remove white blood cells, which constitute less than 1% by weight of whole blood. In particular, when blood cells are stored for a longer period of time, white blood cells may deteriorate and release cytokines, which may if included in a final formulation may cause adverse reactions upon administration. Removal of white blood cells may be achieved by filtration, for example, filtration of whole blood prior to centrifugation, using a leukodepletion filter, such as an IMUGARD^{®} III-RC filter. Red blood cell samples are generally preferably maintained at about 2 ºC to about 8 ºC and at this temperature may be preserved for 30 to 40 days, or can be stored for longer periods of time at temperatures of -20 ºC or lower.

Red blood cells can also be isolated from whole blood by diafiltration using an isotonic solution, having a pH and osmolarity which preserves the integrity of the erythrocyte cellular membrane, for example, a sodium citrate (about 6.0 g/l) and sodium chloride (about 8.0 g/l) solution having an osmolarity of 285 - 315 mOsm. Acceptable diafiltration filters that can be used in accordance herewith include microporous membranes which substantially separate erythrocytes from smaller components, for example, a modified polyethersulfone hollow fiber tangential flow filtration membrane obtainable from Spectrum^{®} Labs Inc. The isotonic solution can be added in batches or continuously, typically approximately at the same rate at which filtrate is lost. During this step, components of the blood solution smaller than red blood cells, generally the plasma portion of the blood, including extracellular blood proteins, e.g. antibodies and serum albumins, are separated as filtrate from the red blood cells, which are retained and continuously or batch-wise added to the isotonic solution. Volumes of isotonic solution used may vary and may for example be at least 2x, 3x, 4x, 5x, 6x or 7x the volume of blood solution. In some embodiments, sufficient volumes of isotonic solution are used to remove at least from about 90% to 95% (mole/mole) of blood plasma proteins and obtain a substantially pure red blood cell preparation, for example, a red blood cell preparation that is at least about 90%, at least about 95%, or at least about 99% pure.

The techniques used to isolate red blood cells from blood and to obtain a red blood cell preparation may be as desired, and include any techniques known to the art, including, further, for example, the methods for isolating red blood cells are described in U.S. Patent 5,955,581.

In order to isolate hemoglobin from isolated red blood cells, isolated red blood cells can be lysed. Any technique for lysing red blood cells known to the art may be used, including any mechanical lysis technique, chemical, or physicochemical fluid tonicity lysis technique, provided however that such technique does not substantially negatively affect the ability of hemoglobin to transport and release oxygen.

In one aspect, the isolated red blood cells can be lysed by subjecting the red blood cells to a hypotonic shock to obtain a red blood cell lysate. Suitable hypotonic solutions that may be used in accordance herewith include, for example, a phosphate buffer 3.75 mM, pH 7.2 or water which may be mixed with the red blood cells, and the mixture may be incubated on ice, for example, for 1 hour to obtain a lysed red blood cell preparation. Subsequently, a hemoglobin fraction can be obtained from the red blood cell lysate using a variety of suitable protein purification techniques, including chromatographic separation techniques which include absorption-based methods, involving initial retention of hemoglobin on solid chromatographic media, and thereafter solvent based elution, or flow-through based techniques, involving retention of impurities and flow through of hemoglobin. Other chromatography-based techniques include affinity chromatography and high-performance liquid-based chromatography (HPLC). Chromatographic techniques to isolate hemoglobin and obtain an isolated hemoglobin preparation that can be used further include, for example, those described in U.S. Patent 5,691,453, Andrade et al., Int. J. Biol. Macromol. 2004, 34: 233-240, and Lu et al., Artif. Cells Blood Substit. Immobil. Biotechnol. 2004, 32: 2004. Alternatively, in order to isolate hemoglobin, the red blood cell lysates may be subjected to non-chromatography-based purification techniques, including, for example, aqueous phase extraction, membrane based micro- and ultrafiltration techniques and tangential flow-based techniques. Ultrafiltration based techniques that can be used include those described by Feins et al. 2005, J. Membr. Sci 248: 137-148. Tangential flow-based techniques that can be used include those described for example by Palmer et al., 2009, in Biotechnol. Progr. 2009, 26 (1) 189-199 and Elmer et al., Biotechnol. Progr. 2009, 25(5) 1402-1410. Two-phase aqueous extraction techniques that can be used to obtain hemoglobin include those described in U.S. Patent 5,407,579.

Using the foregoing techniques, or combinations or modifications thereof, or other suitable techniques, in some embodiments, substantially pure hemoglobin preparations can be obtained, including, for example, preparations which are at least about 80%, 85%, 90%, 95%, 96%, 97%, 98%, or 99% pure.

The hemoglobin preparations obtained using the foregoing techniques, can be used to prepare HBOC preparations, including polymerized HBOC preparations, for example, by cross-linking of hemoglobin molecules, or by chemically modifying hemoglobin molecules. Cross-linking of hemoglobin molecules generally involves the use of cross-linking agents to cause linkage between amino acid residues of multiple hemoglobin molecules, and form intermolecular bonds between hemoglobin molecules. Thus, for example polyaldehydes at final concentrations of between about 0.15 g/l to 20g/l may be used as cross-linking agents. For example, glutaraldehyde at a final concentration of from about 0.15 g/l to about 2 g/l, or succinaldehyde at a final concentration of about 0.15 g/l to about 1.7 g/l may be used to cross-link hemoglobin molecules. Examples of polymerized hemoglobin molecules and techniques for preparing the same are described, for example, in U.S. Patent 5,955,581; U.S. Patent 5,895,810; U.S. Patent Application US2012/0028899 and PCT Patent Applications PCT/CA2017/051111 and PCT/US2018/042497. Examples of chemically modified hemoglobin molecules and techniques for preparing the same include, for example, polysaccharide conjugated hemoglobin (see: *e.g.* PCT Patent Application PCT/CA99/00260), polyalkylene oxide-conjugated hemoglobin (see: *e.g.* U.S. Patent 5,650,388) and polyethylene glycol (PEG) conjugated hemoglobin (see: *e.g.* U.S. Patent 5,750,725, U.S. Patent 7,144,989 and U.S. Patent 8,609,815).

In one embodiment, the HBOC preparation may comprise hyperpolymerized hemoglobin molecules. Hyperpolymerized hemoglobin molecules may have a molecular mass of at least 1,600 kDa and have an oxygen carrying capacity of 11g/dL [Hb], and may exhibit limited or no extravasation (*i.e*. leakage from the blood vessel. Without wishing to be bound by theory, it is thought that smaller sized HBOC molecules may extravasate and bind endothelial nitric oxide (NO) (also known as endothelial-derived relaxing factor), thus reducing the NO concentration, which in turn causes vascular constriction, a reduction in blood flow, and increase in blood pressure.

In some embodiments, an HBOC preparation that may be used in accordance herewith can be a low ionic solute HBOC preparation. The term "low ionic solute HBOC preparation" herein refers to an HBOC preparation comprising an ionic soluble electrolyte concentration that is substantially lower than the physiological soluble electrolyte concentration present in human blood plasma. In this respect, physiological levels of ionic soluble electrolytes are from about 135 to about 145 milliequivalent (mEq/L) sodium ions (Na⁺), from about 3.5 mEq/L to about 5.5 mEq/L potassium ions (K⁺) ions, from about 4.6 mEq/L to about 5.5 mEq/L calcium ions (Ca²⁺), from about 1.5 mEq/L to about 2.5 mEq/L magnesium ions (Mg²⁺) ions, from about 96 mEq/L to about 106 mEq/L chloride ions (CI-) ions, from about 24 mEq/L to about 30 mEq/L bicarbonate ions (HCO₃²⁻), and from about 2.5 mEq/L to about 4.5 mEq/L hydrogen phosphate ions (HPO₄²⁻). In example embodiments, a low ionic solute HBOC preparation can be an HBOC preparation comprising no more than about 27 milliequivalent (mEq/L) sodium ions (Na⁺), no more than about 0.7 mEq/L potassium ions (K⁺) ions, no more than about 0.92 mEq/L calcium ions (Ca²⁺), no more than about 0.3 mEq/L magnesium ions (Mg²⁺) ions, no more than about 19.2 mEq/L chloride ions (CI-) ions, no more than 4.8 mEq/L bicarbonate ions (HCO₃²⁻), and no more than about 0.5 mEq/L hydrogen phosphate ions (HPO₄²⁻); or no more than about 13.5 mEq/L sodium ions (Na⁺), no more than about 0.35 mEq/L potassium ions (K⁺) ions, no more than about 0.46 mEq/L calcium ions (Ca²⁺), no more than about 0.15 mEq/L magnesium ions (Mg²⁺) ions, no more than about 9.6 mEq/L chloride ions (CI-) ions, no more than 2.4 mEq/L bicarbonate ions (HCO₃²⁻), and no more than about 0.25 mEq/L hydrogen phosphate ions (HPO₄²⁻); or no more than about 6.75 mEq/L sodium ions (Na⁺), no more than about 0.175 mEq/L potassium ions (K⁺) ions, no more than about 0.23 mEq/L calcium ions (Ca²⁺), no more than about 0.075 mEq/L magnesium ions (Mg²⁺) ions, no more than about 4.8 mEq/L chloride ions (CI-) ions, no more than 1.2 mEq/L bicarbonate ions (HCO₃²⁻), and no more than about 0.125 mEq/L hydrogen phosphate ions (HPO₄²⁻).

In general, a low ionic solute HBOC preparation can be prepared by starting with a an HBOC preparation comprising physiological concentrations of ionic solute, and exchange the diluent or excipient with a low ionic solute buffer. This can be achieved, for example, by dialysis or diafiltration using a low ionic solute buffer, or using any other suitable technique known to the art.

In some embodiments, the HBOC preparation can be a liquid preparation, or can be prepared to be a liquid formulation, for example, by adding a pharmaceutically acceptable diluent or excipient, such as a saline solution, comprising physiological concentrations of ionic solute or low ionic solute and mixing the HBOC preparation and diluent to dilute the HBOC preparation. In this manner, in preferred embodiments, a liquid HBOC preparation can be prepared to constitute an amount of HBOC from about 10% to about 90% by weight, and an amount of diluent, so that the balance by weight (*i.e.* 90% to 10% by weight) constitutes the diluent.

In some embodiments, the HBOC preparation can be a dried HBOC preparation. In general, in order to obtain a dried HBOC preparation an HBOC preparation in a liquid state may be treated to transform the liquid HBOC preparation into a particulate HBOC preparation. A dried HBOC preparation in accordance herewith can be an HBOC preparation having a moisture content of less than about 10% (w/w) or less, for example, from about 1% (w/w) to about 10% (w/w), or from about 1% (w/w) to about 8% (w/w), or from about 1% (w/w) to about 5% (w/w), or from or from about 1% (w/w) to about 3% (w/w), or from about 1% (w/w) to about 2% (w/w). In this respect a dried HBOC preparation can be prepared, for example, by spray drying or freeze drying a liquid HBOC preparation, using techniques known to those of skill in the art, including techniques substantially similar to the techniques for obtaining a dried blood plasma preparation as hereinbefore described.

Blood plasma preparations, red blood cell preparations, and hemoglobin preparations can also be obtained from a commercial supplier. Commercial blood plasma suppliers include Sigma-Aldrich^{®}, St Louis (MO). Commercial hemoglobin suppliers include Thomas Scientific, Inc. Swedesboro (NJ) and Sigma Aldrich^{®}. Commercial red blood cell suppliers include blood banks, such as the Red Cross.

In accordance with certain aspects of the present disclosure, liquid or dried blood plasma preparations and liquid or dried HBOC preparations can be provided. Example embodiments of the preparation of pharmaceutical formulations using the blood plasma preparations and the HBOC preparations are described.

In one example embodiment, a blood plasma preparation and an HBOC preparation can be contacted with one another and mixed until a substantially homogenous liquid mixture is obtained, for example, by admixing the two preparations in a suitable mixing vessel, for example a beaker, flask, or bottle, preferably at a temperature of 2 ºC to about 8 ºC, by gentle mixing or stirring, using, for example, a magnetic stirrer, or other mechanical stirring device. The thus constituted liquid mixture comprising blood plasma and HBOC, in the context of the present disclosure, is referred to herein as an "oxygenation complement". It is noted that the oxygenation complement can aide and complement existing oxygenation constituents in a subject's circulatory system upon administration thereof to the subject. It is further noted that the term "oxygen complement" herein additionally can also refer to the more or less contemporaneous administration of a separate blood plasma formulation and HBOC formulation, where such will be explicitly clear from the context.

The relative amounts of the HBOC preparation and blood plasma preparation constituting the oxygenation complement may vary. Thus, in example embodiments, the oxygenation complement can comprise an HBOC quantity of from about 5% to about 95% by weight, and a blood plasma quantity of from about 5% to about 95% by weight, wherein the HBOC and blood plasma quantities are selected such that they together constitute less than 100% by weight of the oxygenation complement, and wherein further the balance by weight of the oxygenation complement constitutes, or substantially constitutes a pharmaceutically acceptable diluent, notably a diluent included when the HBOC and blood plasma preparations were prepared.

Thus, for example, the oxygenation complement can be prepared by selecting HBOC and blood plasma quantities such that the oxygenation complement comprises an HBOC quantity of about 5% by weight, and a blood plasma quantity of about 5% to about 90% by weight; or an HBOC quantity of about 10% by weight, and a blood plasma quantity of about 5% to about 85% by weight; or an HBOC quantity of about 15% by weight, and a blood plasma quantity of about 5% to about 80% by weight; or an HBOC quantity of about 20% by weight, and a blood plasma quantity of about 5% to about 75% by weight; or an HBOC quantity of about 25% by weight, and a blood plasma quantity of about 5% to about 70% by weight; or an HBOC quantity of about 30% by weight, and a blood plasma quantity of about 5% to about 65% by weight; or an HBOC quantity of about 35% by weight, and a blood plasma quantity of about 5% to about 60% by weight; or an HBOC quantity of about 40% by weight, and a blood plasma quantity of about 5% to about 55% by weight; or an HBOC quantity of about 45% by weight, and a blood plasma quantity of about 5% to about 55% by weight; or an HBOC quantity of about 50% by weight, and a blood plasma quantity of about 5% to about 45% by weight; or an HBOC quantity of about 55% by weight, and a blood plasma quantity of about 5% to about 40% by weight; or an HBOC quantity of about 60% by weight, and a blood plasma quantity of about 5% to about 35% by weight; or an HBOC quantity of about 65% by weight, and a blood plasma quantity of about 5% to about 30% by weight; or an HBOC quantity of about 70% by weight, and a blood plasma quantity of about 5% to about 25% by weight; or an HBOC quantity of about 75% by weight, and a blood plasma quantity of about 5% to about 20% by weight; or an HBOC quantity of about 80% by weight, and a blood plasma quantity of about 5% to about 15% by weight; or an HBOC quantity of about 85% by weight, and a blood plasma quantity of about 5% to about 10% by weight; or an HBOC quantity of about 90% by weight, and a blood plasma quantity of about 5% by weight; wherein in each of the foregoing selected example HBOC and blood plasma quantities, the balance by weight of the oxygenation complement constitutes, or substantially constitutes, a pharmaceutically acceptable diluent, *i.e.* from about 5% to about 90% by weight of diluent.

Thus, for example, the oxygenation complement can be prepared by selecting HBOC and blood plasma quantities such that the oxygenation complement comprises a blood plasma quantity of about 5% by weight, and an HBOC quantity of about 5% to about 90% by weight; or a blood plasma quantity of about 10% by weight, and an HBOC quantity of about 5% to about 85% by weight; or a blood plasma quantity of about 15% by weight, and an HBOC quantity of about 5% to about 80% by weight; or a blood plasma quantity of about 20% by weight, and an HBOC quantity of about 5% to about 75% by weight; or a blood plasma quantity of about 25% by weight, and a an HBOC quantity of about 5% to about 70% by weight; or a blood plasma quantity of about 30% by weight, and an HBOC quantity of about 5% to about 65% by weight; or a blood plasma quantity of about 35% by weight, and an HBOC quantity of about 5% to about 60% by weight; or a blood plasma quantity of about 40% by weight, and an HBOC quantity of about 5% to about 55% by weight; or a blood plasma quantity of about 45% by weight, and an HBOC quantity of about 5% to about 55% by weight; or a blood plasma quantity of about 50% by weight, and an HBOC quantity of about 5% to about 45% by weight; or a blood plasma quantity of about 55% by weight, and a HBOC quantity of about 5% to about 40% by weight; or a blood plasma quantity of about 60% by weight, and an HBOC quantity of about 5% to about 35% by weight; or a blood plasma quantity of about 65% by weight, and an HBOC quantity of about 5% to about 30% by weight; or a blood plasma quantity of about 70% by weight, and an quantity HBOC of about 5% to about 25% by weight; or a blood plasma quantity of about 75% by weight, and an HBOC quantity of about 5% to about 20% by weight; or a quantity of blood plasma of about 80% by weight, and an HBOC quantity of about 5% to about 15% by weight; or a blood plasma quantity of about 85% by weight, and an HBOC quantity of about 5% to about 10% by weight; or a blood plasma quantity of about 90% by weight, and an HBOC quantity of about 5% by weight; wherein in each of the foregoing selected example HBOC and blood plasma quantities, the balance by weight of the oxygenation complement constitutes, or substantially constitutes, a pharmaceutically acceptable diluent, *i.e.* from about 5% to about 90% by weight of diluent.

Furthermore, by way of non-limiting example only, an oxygenation complement can be constituted to contain an HBOC quantity of 10% by weight, a blood plasma quantity of 35% by weight, the balance by weight (*i.e.* 55%) being constituted by diluent. Such an oxygenation complement may be prepared, for example, by mixing 50% by weight of an HBOC preparation containing an HBOC quantity of 20% by weight and a quantity of diluent of 80% by weight diluent, with 50% by weight of a blood plasma preparation containing a blood plasma quantity of 70% by weight and a quantity of diluent of 30% diluent by weight of diluent.

It is noted that in embodiments hereof where a dried blood plasma preparation (having a moisture content of e.g. from about 1% (w/w) to about 10%(w/w)) is used in order to formulate the oxygenation complement, due to the low moisture content, it is possible to limit the volume (and thus the weight) of pharmaceutical formulations prepared using dried blood plasma. Such limitation of volume (and weight) can be particularly beneficial when the pharmaceutical formulations of the present disclosure are used in battlefield operations, or other operations, for example traffic accident medical emergency operations, where transport or storage of compact, light-weight medical supplies is deemed to be advantageous, for example, to simplify operational logistics or limit transport costs. In one embodiment, in order to prepare a liquid pharmaceutical formulation for administration using a dried blood plasma preparation, a liquid HBOC preparation can be used to reconstitute the dried blood plasma preparation, by mixing the dried blood plasma preparation with the liquid HBOC preparation.

It is noted that since in a dried blood plasma concentrate the endogenous plasma ionic electrolytes are concentrated above physiological concentrations, and may for example, be 5x or 10x of physiological of the physiological concentration, in order to realize a reduction in volume (and weight) of the pharmaceutical formulation, the HBOC preparation is preferably a low ionic solute HBOC preparation, so that upon mixing of the HBOC preparation and the dried blood plasma concentrate, a pharmaceutical formulation comprising about physiological levels of ionic electrolytes is formed. Physiological levels of ionic electrolytes in this respect are from about 135 to about 145 milliequivalent (mEq/L) sodium ions (Na⁺), from about 3.5 mEq/L to about 5.5 mEq/L potassium ions (K⁺) ions, from about 4.6 mEq/L to about 5.5 mEq/L calcium ions (Ca²⁺), from about 1.5 mEq/L to about 2.5 mEq/L magnesium ions (Mg²⁺) ions, from about 96 mEq/L to about 106 mEq/L chloride ions (CI-) ions, from about 24 mEq/L to about 30 mEq/L bicarbonate ions (HCO₃²⁻), and from about 2.5 mEq/L to about 4.5 mEq/L hydrogen phosphate ions (HPO₄²⁻). Thus, for example, if the dried blood concentrate comprises endogenous plasma electrolytes at a concentration of about 9x above physiological concentration, a pharmaceutical formulation may be prepared by mixing about 1x of a dried blood plasma concentrate with about 9x of an HBOC preparation comprising from about 13.5 to about 14.5 milliequivalent (mEq/L) sodium ions (Na⁺), from about 0.35 mEq/L to about 0.55 mEq/L potassium ions (K⁺) ions, from about 0.46 mEq/L to about 0.55 mEq/L calcium ions (Ca²⁺), from about 0.15 mEq/L to about 0.25 mEq/L magnesium ions (Mg²⁺) ions, from about 9.6 mEq/L to about 10.6 mEq/L chloride ions (CI-) ions, from about 2.4 mEq/L to about 3 mEq/L bicarbonate ions (HCO₃²⁻), and from about 0.25 mEq/L to about 0.45 mEq/L hydrogen phosphate ions (HPO₄²⁻).

The oxygenation complement can be contacted with one or more additional pharmaceutically acceptable ingredients, in particular, a pharmaceutically acceptable carrier, diluent or auxiliary agent, to formulate a pharmaceutical formulation comprising the oxygenation complement and a pharmaceutically acceptable carrier, diluent or auxiliary agent.

In one aspect, the oxygenation complement can be contacted with a pharmaceutically acceptable carrier, diluent or auxiliary agent and mixed until a substantially homogenous liquid mixture is obtained. In this regard, the oxygenation complement and the pharmaceutically acceptable carrier, diluent or auxiliary agent can be admixed in a suitable mixing vessel, for example, a beaker, flask, or bottle, once again, preferably at a temperature of 2 ºC to about 8 ºC, by gentle stirring, using, for example, a magnetic stirrer, or other mechanical stirring device.

Suitable pharmaceutically acceptable carriers include liquids including water, Lactated Ringer's solution, modified Lactated Ringer's solution that includes an antioxidant such as N-acetyl cysteine, saline solutions, such phosphate buffered saline solutions, hypertonic and isotonic solutions, such as hypertonic or hypotonic sodium chloride, and emulsions, such as oil/water emulsions.

Suitable pharmaceutically acceptable diluents that may be used in accordance herewith include water, buffers, for example, Ringer's solution, Hank's solution and physiologically buffered saline solutions, for example, phosphate buffered saline solutions, and Lactated Ringer's solution, modified Lactated Ringer's solution that includes an antioxidant such as N-acetyl cysteine.

The pharmaceutical formulations of the present disclosure may further be formulated to include auxiliary substances, such as preservatives, anti-oxidant, antimicrobial agents, binders, emulsifying agents, pH buffering agents, including amino acids which may function as buffering agents, such as glutamic acid, aspartic acid, histidine, arginine, salts, or adjuvants. These auxiliary substances are generally included in small amounts, together constituting 10% or less, or 5% or less, or 2.5% or less by weight of the pharmaceutical formulations of the present disclosure.

In one embodiment, an auxiliary substance that may be included in the pharmaceutical formulations of the present disclosure are blood platelets. In general, platelets may be included in the pharmaceutical formulations to facilitate blood clotting and hemostasis. Blood platelets may be prepared from human or animal blood using techniques known to the art, including, for example techniques, described in PCT Patent Application PCT/US93/04142 and U.S. Patent Application having Publication No US2021/0023140, which are included herein by reference. Furthermore, the platelets may be synthetic platelets, such as for example described in U.S. Patent 10,426,820. Platelets may be provided in concentrated or dry form, for example, spray dried or lyophilized form, for use in the formulation of the pharmaceutical formulations of the disclosure. Platelets may also be commercially purchased, for example, from Cellero^{Tm} (Lowell, MA). Generally, the pharmaceutical formulations of the present disclosure are formulated to include platelets in an amount of about 5% or less, about 4% or less, about 3% or less, or about 2% or less, or about 1% or less, including for example from about 0.5% to about 5%, from about 0.5% to about 2.5%, or from about 0.5% to about 1% by weight of the pharmaceutical formulations.

The relative amounts of oxygenation complement and the pharmaceutically acceptable carrier, diluent or auxiliary agent may vary. In one embodiment, for example, the pharmaceutical formulation can comprise or consist of up to 50%, 60%, 70%, 80%, 90%, or 95% by weight of the oxygenation complement, the oxygenation complement comprising a quantity of from about 5% to about 95% by weight, and a quantity of from about 5% to about 95% by weight of undiluted blood plasma, the quantities of HBOC and undiluted blood plasma selected such that they together constitute less than 100% by weight of the oxygenation complement, the balance by weight of the oxygenation complement being constituted by a pharmaceutically acceptable diluent; and the balance by weight of the pharmaceutical formulation being substantially constituted, or constituted by a pharmaceutically acceptable carrier, diluent or auxiliary agent.

By way of non-limiting example only, an oxygenation complement can be constituted to contain an HBOC quantity of 5% by weight, a blood plasma quantity of 45% by weight, the balance by weight (*i.e.* 50%) being constituted by a diluent. Such an oxygenation complement may, for example, be prepared by mixing 50% by weight of an HBOC preparation containing an HBOC quantity of 10% by weight HBOC and 90% by weight diluent, with 50% by weight of a blood plasma preparation containing a blood plasma quantity of 90% by weight and 10% by weight diluent. A pharmaceutical formulation may be formulated to contain 70% by weight of the oxygenation constituent, and the balance by weight (*i.e.* 30%) containing 28% by weight of a pharmaceutically acceptable diluent, and 2% by weight of a pharmaceutically acceptable auxiliary agent. The same pharmaceutical formulation contains 3.5% by weight HBOC and 31.5% by weight undiluted blood plasma.

It is noted that to prepare the liquid pharmaceutical formulations of the present disclosure, in different embodiments, the order in which the various constituent compounds of the pharmaceutical formulations are mixed may be varied, as desired. For example, it is also possible to initially select and mix a pharmaceutically acceptable carrier, diluent or auxiliary agent with an HBOC preparation, and thereafter contact the obtained mixture with a blood plasma preparation to form a pharmaceutical formulation comprising an oxygenation complement comprising blood plasma and HBOC; or it is possible to initially select and mix a pharmaceutically acceptable carrier, diluent or auxiliary agent with a blood plasma preparation, and thereafter contact the obtained mixture with an HBOC preparation to form a pharmaceutical formulation comprising an oxygenation complement comprising blood plasma and HBOC.

It is further noted that he liquid pharmaceutical formulations of the present disclosure can be said to be protein soluble formulations, *i.e.* the proteins contained therein are contained therein in a dissolved state.

Thus, to briefly recap, in accordance herewith, in an aspect, preparations comprising HBOC may be prepared and preparations comprising blood plasma may be prepared. These preparations may be used to prepare an oxygenation complement. The oxygenation complement may be used to formulate a pharmaceutical formulation, notably a pharmaceutical formulation containing an effective amount of the oxygenation complement to administer the pharmaceutical formulation to a subject in need thereof to treat or prevent hemorrhagic shock.

It will be clear from the foregoing that in one aspect, the present disclosure further provides, in an embodiment, a method of preparing a liquid pharmaceutical formulation for the prevention or treatment of hemorrhagic shock, the method comprising:
mixing
   (i) a hemoglobin-based oxygen carrier (HBOC) preparation; and
   (ii) a blood plasma preparation;
to thereby constitute an oxygenation complement and formulate a liquid pharmaceutical formulation.

It is noted that in some embodiments the pharmaceutical formulation may be formulated as a finished formulation in a manufacturing facility suitable for the manufacture of pharmaceutical grade materials, and subsequently, in a single package, be transported and/or stored until use for administration to a subject in need thereof is desired.

In other embodiments, the HBOC preparation and the blood plasma preparation may be manufactured and packaged in two separate packages, and the liquid formulation may be prepared just prior to the administration thereof to a subject in need thereof, for example, less than 6hrs, less than 5 hrs, less than 4 hrs, less than 3 hours, less than 2 hours or less than 1 hour prior to administration.

Thus, in another aspect, the present disclosure provides a pharmaceutical kit or package comprising (i) a first package comprising a hemoglobin-based oxygen carrier (HBOC) preparation and (ii) a second package comprising a blood plasma preparation, wherein upon mixing of the HBOC preparation and the blood plasma preparation a liquid pharmaceutical formulation to prevent or treat hemorrhagic shock is constituted, together with instructions to prepare the liquid pharmaceutical formulation.

The pharmaceutical package and the first and second package may be selected from a wide variety of containers, and can, for example, be a bottle, a tube, a bag, a pouch, a sachet, a syringe or the like, manufactured to be suitable to contain, store, transport and dispense medicinal products. Thus, such containers are generally manufactured to limit spoilage by heat, oxygen, microbial contamination, moisture, and so on, and to facilitate the formulation of the liquid pharmaceutical formulations of the present disclosure.

In some embodiments, the pharmaceutical package may further comprise (iii) a third package comprising a pharmaceutically acceptable carrier, diluent or auxiliary agent for mixing with the HBOC preparation and the blood plasma preparation to thereby form the liquid pharmaceutical formulation.

A light-weight pharmaceutical package may include an HBOC preparation and/or blood plasma preparation wherein in the HBOC preparation and/or the blood plasma preparation are dried preparations, having a moisture content from about 1% (w/w) to about 10% (w/w). As will be appreciated by those of skill in the art, in embodiments in which both the HBOC preparation and the blood plasma preparation are dried preparations, in order to prepare a pharmaceutical formulation it will be necessary to combine the dried HBOC preparation and the dried blood plasma preparation with a pharmaceutically acceptable diluent prior to administration.

Further included in the package may be instructions for preparing the liquid pharmaceutical formulations, and the package may further include instructions for administration (e.g. printed on paper and provided directly with the package), and/or a reference may be provided for on-line access to the instructions, all of which are intended to be included herein. The instructions may additionally comprise further information regarding the products and its use, e.g. safety information. The pharmaceutical packages may further include devices, such as a needles and syringes, for the intravenous administration of the liquid pharmaceutical formulations.

In another aspect, the present disclosure provides, in one embodiment, a use of a hemoglobin-based oxygen carrier and blood plasma to prepare an oxygenation complement to formulate a protein-soluble liquid pharmaceutical formulation.

As hereinbefore noted, the pharmaceutical formulations of the present disclosure may be used to prevent or treat hemorrhagic shock. Hence, in another aspect, the present disclosure provides, in at least one embodiment, a method for preventing or treating hemorrhagic shock, the method comprising intravenously administering to a subject in need thereof an effective amount of an oxygenation complement comprising a hemoglobin-based oxygen carrier (HBOC) and blood plasma, in an effective amount to prevent or treat hemorrhagic shock in the subject.

In some embodiments, the oxygenation complement can be administered to the subject by more or less contemporaneous consecutive administration of a liquid HBOC formulation and a liquid blood plasma formulation, wherein the liquid HBOC formulation is substantially free of blood plasma, and wherein the liquid blood plasma formulation is substantially free of HBOC.

In some embodiments, the oxygenation complement can be administered to the subject by consecutive administration of the HBOC formulation and the blood plasma formulation, wherein the HBOC formulation is administered prior to the blood plasma formulation.

In some embodiments, the HBOC formulation and the blood plasma formulation can be administered no more than about 12 hours apart from each other, e.g. no more than 6 hours apart, no more than 3 hours apart, no more than 2 hours apart, or no more than 1 hour apart from each other.

In some embodiments, the oxygenation complement can be administered to the subject by consecutive delivery of the HBOC formulation and the blood plasma formulation, wherein the blood plasma formulation is administered prior to the HBOC formulation.

In some embodiments, the oxygenation complement can be administered by administering a liquid pharmaceutical formulation having been prepared by mixing an HBOC preparation and a blood plasma preparation.

In some embodiments, a pharmaceutical formulation comprising blood platelets can be co-administered with the oxygenation complement. By the term "co-administered" it is meant that the pharmaceutical formulation comprising blood platelets is administered separately but more or less contemporaneously with the oxygenation complement, for example about 1 hour or less, about 30 minutes or less, or about 15 or less prior to or following the administration of oxygenation complement.

In another aspect, the present disclosure provides, in an embodiment, a use of a liquid pharmaceutical formulation to prevent or treat hemorrhagic shock, the liquid formulation comprising an oxygenation complement comprising a hemoglobin-based oxygen carrier (HBOC) and blood plasma and a pharmaceutically acceptable carrier, diluent or auxiliary agent.

In another aspect, the present disclosure provides, in an embodiment, a liquid pharmaceutical formulation for use in preventing or treating hemorrhagic shock, the formulation comprising an oxygenation complement comprising a hemoglobin-based oxygen carrier (HBOC) and blood plasma and a pharmaceutically acceptable carrier, diluent or auxiliary agent.

In another aspect, the present disclosure provides, in an embodiment, a use of a liquid pharmaceutical formulation in the manufacture of a medicament for use in preventing or treating hemorrhagic shock, the formulation comprising an oxygenation complement comprising a hemoglobin-based oxygen carrier (HBOC) and blood plasma or blood plasma constituents and a pharmaceutically acceptable carrier, diluent or auxiliary agent.

The liquid pharmaceutical formulations of the present disclosure may be administered to a subject in need thereof and are generally administered intravenously to prevent or treat hemorrhagic shock. The cause of the hemorrhagic shock may vary and includes trauma, including blunt trauma or penetrating trauma, or non-traumatic bleeding. For the purposes of the present disclosure, the cause of hemorrhagic shock is of no particular importance, and the liquid pharmaceutical formulations may be used to prevent or treat a subject having experienced, or expecting to be experiencing any hemorrhagic shock, irrespective of its cause. As is known to those of skill in the art, in addition to substantial blood loss, other clinical diagnostic symptoms of hemorrhagic shock include tachycardia, tachypnea, a narrowing pulse and cooling extremities. Any or all of these symptoms may be observed in a subject and given due reasonable medical consideration prior to the administration of the pharmaceutical formulations of the present disclosure to a subject.

In accordance with an aspect hereof, an effective amount of the liquid formulations is administered to a subject. The administered dose of the pharmaceutical formulations may vary, for example, depending on the severity of the hemorrhagic shock condition, or the age and size of the subject. For example, when the hemorrhagic shock is estimated to be caused by a blood loss of 15% of the total blood volume, a volume of the liquid pharmaceutical formulations approximately equal to about 15% may be administered, or when the hemorrhagic shock is estimated to be caused by a blood loss of 20% of the total blood volume, a volume of the pharmaceutical formulations approximately equal to 20% may be administered, and so forth. A single or multiple doses of the liquid pharmaceutical formulation may be administered to a subject. In particular, when upon the administration of a first dose of a pharmaceutical formulation, bleeding is not stopped, one or more additional doses of the liquid pharmaceutical formulation may be administered to the subject.

Upon administration of the pharmaceutical formulations of the present disclosure, the symptoms of hemorrhagic shock may improve. Thus, for example, upon administration of the pharmaceutical formulations of the present disclosure hemostasis may be attained. Furthermore, clinical diagnostic symptoms of hemorrhagic shock, including the aforementioned symptoms, may disappear.

As now can be appreciated, pharmaceutical formulations in accordance with the present disclosure pharmaceutical formulations can be formulated, including pharmaceutical formulations comprising an oxygenation complement comprising a hemoglobin-based oxygen carrier (HBOC) and blood plasma. The pharmaceutical formulations can be used to prevent or treat hemorrhagic shock.

The above-described example embodiments of the present disclosure are intended to be illustrative only and in no way limiting. The described embodiments are susceptible to many modifications of composition, details and order of operation. The disclosure, rather, is intended to encompass all such modifications within its scope, as defined by the claims, which should be given a broad interpretation consistent with the description as a whole.

### EXAMPLES

Hereinafter are provided examples of further specific embodiments for performing the methods of the present disclosure, as well as embodiments representing the compositions of the present disclosure. The examples are provided for illustrative purposes only and are not intended to limit the scope of the present disclosure in any way.

### Example 1 - Preparation of an HBOC preparation ("OxyBridge")

Packed red blood cells were obtained from an authorized distributor and were washed via 6 diafiltration exchanges of normal saline across a 0.45 µM hollow fiber filter and then lysed with a further 6 diafiltration exchanges of purified water. The filtrate fraction containing proteins was then purified with 6 diafiltration exchanges of phosphate buffer across a 500 kDa hollow fiber filter, and then the collected protein in the filtrate was further washed with 6 diafiltration exchanges of phosphate buffer and n-acetyl cysteine across a 10 kDa hollow fiber filter, keeping the retentate. During the last step, the solution was concurrently deoxygenated with a gas exchange cartridge and nitrogen. The resulting protein solution (2 g/dL) was reacted with glutaraldehyde at a final concentration of 1.25 g/l) for 3 hours and then reduced with cyanoborohydride (BH₃CN) and was put into a lactated Ringer's solution with n-acetyl cysteine, such that the glutaraldehyde and cyanoborohydride were removed via diafiltration to thereby obtain a low purity blood substitute preparation. The concentration of endogenous non-hemoglobin protein complement in the protein preparation was estimated to be between about 0.2 mole/mole and about 20% mole/mole, based on an SDS gel evaluation. All steps were performed at room temperature. The obtained HBOC preparation is referred to herein as "OxyBridge".

OxyBridge can be said to be a human hemoglobin-based oxygen carrier product of hyperpolymerized human Hb of an average high molecular weight of 1.6 MDa, with a narrow range in polymer size. Other OxyBridge properties include above-average Hb concentration (11 g/dL), low solution colloid osmotic pressure (12 mmHg), and high viscosity (12 cPs). The OxyBridge molecular design acts by virtue of its large size (MW; 1600 kDa vs 64 kDa for a hemoglobin tetramer) and high oxygen carrying capacity (hemoglobin concentration; 11 g/dL). The polymeric nature provides colloidal and viscous properties. OxyBridge is formulated under a deoxygenated atmosphere so that it is not subject to oxidation by ambient oxygen.

### Example 2 - Preparation of a blood plasma preparation

For preparation of blood plasma, whole blood samples containing anticoagulant additives were centrifuged at 2,000 x g for 20 min to separate the plasma layer, which was collected. It is noted that the collected blood plasma can be centrifuged a second time at 2,000 x g for 20 min for increased purity. Alternatively, commercially available fresh frozen plasma or spray dried plasma can be used.

### Example 3 - Preparation of a low electrolyte solute HBOC preparation

For preparation of a low electrolyte HBOC solution, HBOC can be prepared as described in Example 1 and then diafiltered with a low electrolyte buffer for at least 3 exchanges, preferably 6 exchanges, using any filter that allows for diafiltration and has a pore size molecular weight cut off range smaller than the molecular weight of the HBOC. In case a preparation if HBOC polymers is used wherein the polymers have a range of a polymer sizes, a filter can be selected that has a pore size that is less than the molecular weight of the smaller-sized polymers. If a yield loss of HBOC is acceptable, the filter pore size can be selected to have a molecular weight size cut off that may be larger than the smallest polymers in the preparation but still smaller than the average polymer molecular weight. Thus, when using the HBOC preparation of Example 1, a filter may be selected having a pore size of less than 1.6 MDa.

### Example 4 - Formulation of a pharmaceutical formulation comprising blood plasma and HBOC

Blood plasma and HBOC preparations were prepared as described in Examples 3 and 1, respectively, and subsequently 5 ml of the blood plasma preparation and 5 ml of the HBOC preparation were poured into a 10 ml tube with a screw cap. A pharmaceutical formulation was prepared my repeated manual inversion of the screw-capped tube until a homogenous mixture was obtained.

In general, in order to prepare the pharmaceutical formulation for *in vivo* use, the HBOC and blood plasma preparations are physiologically compatible with each other, such that mixing them in any ratio results in a pharmaceutical formulation. Both are active ingredients whose function is at least partially limited by concentration so when a balanced formulation is desired, such as used for the performance of the work described in Example 6, a 1 to 1 mixture is desirable. The pharmaceutical formulation is preferably prepared shortly before use.

### Example 5 - Efficacy of a pharmaceutical formulation comprising blood plasma and HBOC (in vitro)

This example describes an assessment of coagulopathy of pharmaceutical formulations comprising blood plasma and HBOC.

Whole blood (WB) was collected by phlebotomy from six healthy donors under an approved institutional standard operating procedure. Fresh frozen plasma was obtained by centrifugation of whole blood from a different set of ten donors (Group O+), which was pooled and aliquoted prior to storage at -80 °C until the day of testing and rapidly thawed at 37 °C immediately prior to usage. As this was an *in vitro* evaluation, HBOC was exposed to 100% oxygen in a 50 ml conical tube, and oxygen saturation was measured on the RAPIDPoint 500 (Siemens) prior to usage. *In vivo,* HBOC is oxygenated by passage through the lungs.

Samples were prepared to simulate various resuscitation scenarios by mixing WB with additives at different ratios. A total of 18 samples were prepared, with WB used as the control, 10% and 25% volumetric replacements with OxyBridge, fresh frozen plasma (FFP), a 50-50 split of each, or Lactate-Ringers (LR) (as the OxyBridge vehicle control) as primary testing conditions. In a parallel set of samples, WB was pre-diluted with 25% PlasmaLyte (DIL) to simulate a dilutional coagulopathy prior to adding the volumetric replacements listed above. **Table 1** below shows the 18-sample set.

Hemoglobin, hematocrit, and platelet count were determined on the Advia 120 (Siemens). Samples were subjected to shearing over a collagen-coated surface in a microfluidic model of capillary flow to measure the effect on platelet adhesion in the BioFlux 1000 system (Fluxion Biosciences). Following centrifugation, the liquid portion was analyzed for prothrombin time (PT) and fibrinogen concentration on the STA-R Evolution (Stago) as well as thrombin generation on the Calibrated Automated Thrombogram (CAT; Thrombinoscope). The results are shown in **FIGS. 1 - 6****.**

**Table 1: In vitro Test Samples.**

| **Sample** | **WB** | **FFP** | **HBOC** | **LR** | **DIL** |
|---|---|---|---|---|---|
| Control | 100 | | | | |
| 10% FFP | 90 | 10 | | | |
| 10% HBOC | 90 | | 10 | | |
| 10% FFP/HBOC | 90 | 5 | 5 | | |
| 10% LR | 90 | | | 10 | |
| Dil. Control | 75 | | | | 25 |
| Dil. 10% FFP | 65 | 10 | | | 25 |
| Dil. 10% HBOC | 65 | | 10 | | 25 |
| Dil. 10% FFP/HBOC | 65 | | 5 | 5 | 25 |
| Dil. 10% LR | 65 | | | 10 | 25 |
| 25% FFP | 75 | 25 | | | |
| 25% HBOC | 75 | | 25 | | |
| 25% FFP/HBOC | 75 | 12.5 | 12.5 | | |
| 25% LR | 75 | | | 25 | |
| Dil. 25% FFP | 50 | 25 | | | 25 |
| Dil. 25% HBOC | 50 | | 25 | | 25 |
| Dil. 25% FFP/HBOC | 50 | 12.5 | 12.5 | | 25 |
| Dil. 25% LR | 50 | | | 25 | 25 |

As can be seen in **FIGS. 1** - **6****,** no negative impact on coagulation parameters was detected using pharmaceutical formulations comprising blood plasma and HBOC. In particular, no adverse impact was observed on platelet count in samples including HBOC + FFP (**FIG. 3**), no indication of cell lysis in samples including HBOC + FFP (**FIG. 4**), and no indication of impact on fibrinogen concentration in samples including HBOC + FFP (**FIG. 5**). Furthermore, the results in **FIGS. 1** and **2****,** show that in formulations comprising HBOC alone, coagulation parameters (prothrombin time and clot lysis) are diminished. These effects were negated when, in addition to HBOC, FFP was included in the formulations.

### Example 6 - Efficacy of a pharmaceutical formulation comprising blood plasma and HBOC (in vivo)

Three pharmaceutical formulations were tested in a controlled hemorrhage rat study (n=4) for the ability of these formulations to provide resuscitation relief as follows: (i) HBOC (OxyBridge), (ii) Plasma (fresh autologous plasma), and (iii) HBOC (OxyBridge) + Plasma. HBOC (OxyBridge), Plasma, and HBOC (OxyBridge) + Plasma formulations were prepared as described in Examples 1, 3, and 4, respectively. One hour following a variable rate, controlled hemorrhage (50% of the estimated total blood volume, T_{BV}), the pharmaceutical formulations were administered. Resuscitation volume was limited to a single 20% T_{BV} bolus (equivalent to 1 L in humans) to simulate limited resuscitation availability in austere combat theaters. Following resuscitation, a blood sample was collected to assess the coagulation panel using thromboelastometry and hemostasis analyzer. Then 80% of the rat tail was excised, inducing uncontrolled hemorrhage in the animal. This study, in part, was to identify and correlate coagulopathic indicators (*e.g.,* max clot firmness and prothrombin time) with hemostasis and to establish how this resuscitation strategy performed. Fresh blood samples were used for thromboelastometry. Plasma samples were collected and banked to be used in a hemostasis analyzer. Once analyzed, trends associated with prothrombin time and/or D dimer were evaluated with the hemostasis results.

None of the HBOC (OxyBridge)-treated alone animals achieved hemostasis (0/0), but there was also no evidence of fibrinolysis. Plasma-treated animals had a slightly higher success rate of hemostasis at 25% (1/4). However unexpectedly, and importantly, the combination of HBOC (OxyBridge) + Plasma achieved hemostasis in 100% of the animals (4/4).

Therefore in this study, while resuscitation using HBOC (OxyBridge) (0/0) or Plasma (1/4) alone did not provide successful hemostasis in any or most test animals, a combination of the two products (HBOC) (OxyBridge) + Plasma) provided hemostasis in 100% (4/4) of test animals. Furthermore, importantly, these findings show that HBOC (Oxybridge) did not induce fibrinolysis. Furthermore, when administered at a field relevant dose, HBOC (Oxybridge) does not induce dilutional coagulopathy as more traditional plasma expanders have been shown to do.

## Claims

1. A liquid low ionic solute hemoglobin-based oxygen carrier (HBOC) preparation comprising:
an HBOC,
no more than about 27 milliequivalent (mEq/L) sodium ions (Na⁺),
no more than about 0.7 mEq/L potassium ions (K⁺),
no more than about 0.92 mEq/L calcium ions (Ca²⁺),
no more than about 0.3 mEq/L magnesium ions (Mg²⁺),
no more than about 19.2 mEq/L chloride ions (CI-),
no more than 4.8 mEq/L bicarbonate ions (HCO₃²⁻), and
no more than about 0.5 mEq/L hydrogen phosphate ions (HPO₄²⁻).

2. The liquid low ionic solute HBOC preparation according to claim 1, wherein
(i) the liquid low ionic solute HBOC preparation comprises no more than about 13.5 mEq/L sodium ions (Na⁺), no more than about 0.35 mEq/L potassium ions (K⁺), no more than about 0.46 mEq/L calcium ions (Ca²⁺), no more than about 0.15 mEq/L magnesium ions (Mg²⁺), no more than about 9.6 mEq/L chloride ions (Cl-), no more than 2.4 mEq/L bicarbonate ions (HCO₃²⁻), and no more than about 0.25 mEq/L hydrogen phosphate ions (HPO₄²⁻);
optionally wherein the liquid low ionic solute HBOC preparation comprises no more than about 6.75 mEq/L sodium ions (Na⁺), no more than about 0.175 mEq/L potassium ions (K⁺), no more than about 0.23 mEq/L calcium ions (Ca²⁺), no more than about 0.075 mEq/L magnesium ions (Mg²⁺), no more than about 4.8 mEq/L chloride ions (Cl-), no more than 1.2 mEq/L bicarbonate ions (HCO₃²⁻), and no more than about 0.125 mEq/L hydrogen phosphate ions (HPO₄²⁻); and/or
(ii) the liquid low ionic solute HBOC preparation comprises an HBOC quantity of from about 5% to about 50% by weight, the balance of the preparation comprising a pharmaceutically acceptable diluent.

3. A liquid pharmaceutical formulation comprising an oxygenation complement comprising the liquid low ionic solute HBOC preparation according to claim 1 or 2, and blood plasma.

4. A liquid pharmaceutical formulation according to claim 3, wherein the oxygenation complement comprises an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent;
for example wherein the oxygenation complement comprises an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent.

5. A liquid pharmaceutical formulation according to claim 3, wherein the pharmaceutical formulation comprises:
(i) up to about 95% by weight of the oxygenation complement, the oxygenation complement comprising an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and
the balance by weight of the pharmaceutical formulation being constituted by a pharmaceutically acceptable carrier, diluent, or auxiliary agent;
for example, (ii) up to about 95% by weight of the oxygenation complement, the oxygenation complement comprising an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and
the balance by weight of the pharmaceutical formulation being constituted by a pharmaceutically acceptable carrier, diluent, or auxiliary agent.

6. A liquid pharmaceutical formulation according to claim 3, wherein the HBOC comprises polymerized hemoglobin molecules having a molecular mass of at least 1,600 kDa;
or wherein the auxiliary agent comprises blood platelets.

7. An oxygenation complement comprising the liquid low ionic solute HBOC preparation according to claim 1 or 2 and blood plasma for use in a method for preventing or treating hemorrhagic shock, the method comprising intravenously administering to a subject in need thereof the oxygenation complement comprising the liquid low ionic solute HBOC preparation and blood plasma, in an effective amount to prevent or treat hemorrhagic shock in the subject.

8. The oxygenation complement for use in the method for preventing or treating hemorrhagic shock according to claim 7, wherein
(i) the oxygenation complement is administered to the subject by consecutive administration of the liquid low ionic solute HBOC preparation and a liquid blood plasma formulation, wherein the liquid low ionic solute HBOC preparation is substantially free of blood plasma, and wherein the liquid blood plasma formulation is substantially free of HBOC;
(ii) the oxygenation complement is administered to the subject by consecutive administration of the liquid low ionic solute HBOC preparation and the blood plasma formulation, wherein the liquid low ionic solute HBOC preparation is administered prior to the blood plasma formulation;
(iii) the oxygenation complement is administered to the subject by consecutive delivery of the liquid low ionic solute HBOC preparation and the blood plasma formulation, wherein the blood plasma formulation administered prior to the liquid low ionic solute HBOC preparation; or
(iv) the liquid low ionic solute HBOC preparation and the blood plasma formulation are administered to the subject no more than about 6 hours apart from each other; or
(v) the oxygenation complement is administered by administering a liquid pharmaceutical formulation having been prepared by mixing the liquid low ionic solute HBOC preparation and the blood plasma preparation;
optionally wherein the liquid pharmaceutical formulation further comprises a pharmaceutically acceptable carrier, diluent or auxiliary agent.

9. The oxygenation complement for use in the method for preventing or treating hemorrhagic shock according to claim 8, part (v), wherein
(i) the oxygenation complement comprises an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent;
(ii) the liquid pharmaceutical formulation comprises up
to 95% by weight of the oxygenation complement, the oxygenation complement comprising an HBOC quantity of from about 5% to about 95% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and
the balance by weight of the pharmaceutical formulation being constituted by the pharmaceutically acceptable carrier, diluent, or auxiliary agent;
optionally wherein the auxiliary agent comprises blood platelets;
(iii) the oxygenation complement comprises an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; or
(iv) the liquid pharmaceutical formulation comprises
up to 95% by weight of the oxygenation complement, the oxygenation complement comprising an HBOC quantity of from about 5% to about 50% by weight, and a blood plasma quantity of from about 5% to about 95% by weight, the HBOC and blood plasma quantities selected such that they together constitute less than 100% by weight of the oxygenation complement, the balance by weight of the oxygenation complement being constituted by a pharmaceutically acceptable diluent; and
the balance by weight of the pharmaceutical formulation being constituted by the pharmaceutically acceptable carrier, diluent, or auxiliary agent.

10. The oxygenation complement for use in the method for preventing or treating hemorrhagic shock according to any one of claims 7 to 9, wherein
(i) a pharmaceutical formulation comprising blood platelets is co-administered with the oxygenation complement;
(ii) the hemorrhagic shock is caused by a traumatic blood loss in the subject; or
(iii) the hemorrhagic shock is caused by a non-traumatic blood loss in the subject.

11. A pharmaceutical package comprising (i) a first package comprising the liquid low ionic solute HBOC preparation according to claim 1 or 2 and (ii) a second package comprising a blood plasma preparation, and instructions to prepare the liquid pharmaceutical formulation according to claim 3.

12. A pharmaceutical package according to claim 11, wherein the pharmaceutical package further comprises (iii) a third package comprising a pharmaceutically acceptable carrier, diluent or auxiliary agent for mixing with the liquid low ionic solute HBOC preparation and the blood plasma preparation to thereby form the liquid pharmaceutical formulation.

13. A pharmaceutical package according to claim 11, wherein the blood plasma preparation is a dried blood plasma preparation having a moisture content from about 1% (w/w) to about 10% (w/w);
optionally wherein the liquid low ionic solute HBOC preparation can comprise no more than about 13.5 mEq/L sodium ions (Na⁺), no more than about 0.35 mEq/L potassium ions (K⁺), no more than about 0.46 mEq/L calcium ions (Ca²⁺), no more than about 0.15 mEq/L magnesium ions (Mg²⁺), no more than about 9.6 mEq/L chloride ions (Cl-), no more than 2.4 mEq/L bicarbonate ions (HCO₃²⁻), and no more than about 0.25 mEq/L hydrogen phosphate ions (HPO₄²⁻).

14. A method of preparing a liquid pharmaceutical formulation according to any one of claims 3 to 6, the method comprising:
mixing
(i) the liquid low ionic solute HBOC preparation according to claim 1 or 2; and
(ii) a blood plasma preparation;
to thereby constitute an oxygenation complement or formulate a liquid
pharmaceutical formulation.

15. A method of preparing a liquid pharmaceutical formulation according to claim 14, wherein
(a) the blood plasma preparation is a dried blood plasma preparation having a moisture content from about 1% (w/w) to about 10% (w/w),
optionally wherein the liquid low ionic solute HBOC preparation can comprise no more than about 13.5 mEq/L sodium ions (Na⁺), no more than about 0.35 mEq/L potassium ions (K⁺), no more than about 0.46 mEq/L calcium ions (Ca²⁺), no more than about 0.15 mEq/L magnesium ions (Mg2+), no more than about 9.6 mEq/L chloride ions (Cl-), no more than 2.4 mEq/L bicarbonate ions (HCO₃²⁻), and no more than about 0.25 mEq/L hydrogen phosphate ions (HPO₄²⁻);
(b) the method further comprises mixing (iii) a pharmaceutically acceptable carrier, diluent, or auxiliary agent together with the oxygenation complement to thereby form a liquid pharmaceutical formulation; or
(c) the liquid low ionic solute HBOC preparation and the blood plasma preparation are mixed 1 hour or less prior to administration to a subject in need thereof.

## Patentansprüche

1. Flüssige Hämoglobin-basierter-Sauerstoffträger (HBOC)-Zubereitung mit niedrigem Gelöst-Ionengehalt, umfassend:
einen HBOC,
nicht mehr als etwa 27 Milliäquivalente (mEq/L) Natriumionen (Na⁺),
nicht mehr als etwa 0,7 mEq/L Kaliumionen (K⁺),
nicht mehr als etwa 0,92 mEq/L Calciumionen (Ca²⁺),
nicht mehr als etwa 0,3 mEq/L Magnesiumionen (Mg²⁺),
nicht mehr als etwa 19,2 mEq/L Chloridionen (Cl-),
nicht mehr als 4,8 mEq/L Bicarbonationen (HCO₃²⁻), und
nicht mehr als etwa 0,5 mEq/L Hydrogenphosphationen (HPO₄²⁻).

2. Flüssige HBOC-Zubereitung mit niedrigem Gelöst-Ionengehalt nach Anspruch 1, wobei
(i) die flüssige HBOC-Zubereitung mit niedrigem Gelöst-Ionengehalt nicht mehr als etwa 13,5 mEq/L Natriumionen(Na⁺), nicht mehr als etwa 0,35 mEq/L Kaliumionen (K⁺), nicht mehr als etwa 0,46 mEq/L Calciumionen (Ca²⁺), nicht mehr als etwa 0,15 mEq/L Magnesiumionen (Mg²⁺), nicht mehr als etwa 9,6 mEq/L Chloridionen (Cl-), nicht mehr als 2,4 mEq/L Bicarbonationen (HCO₃²⁻) und nicht mehr als etwa 0,25 mEq/L Hydrogenphosphationen (HPO₄²⁻) umfasst;
gegebenenfalls wobei die flüssige HBOC-Zubereitung mit niedrigem Gelöst-Ionengehalt nicht mehr als etwa 6,75 mEq/L Natriumionen(Na⁺), nicht mehr als etwa 0,175 mEq/L Kaliumionen (K⁺), nicht mehr als etwa 0,23 mEq/L Calciumionen (Ca²⁺), nicht mehr als etwa 0,075 mEq/L Magnesiumionen (Mg²⁺), nicht mehr als etwa 4,8 mEq/L Chloridionen (Cl-), nicht mehr als 1,2 mEq/L Bicarbonationen (HCO₃²⁻) und nicht mehr als etwa 0,125 mEq/L Hydrogenphosphationen (HPO₄²⁻) umfasst; und/oder
(ii) die flüssige HBOC-Zubereitung mit niedrigem Gelöst-Ionengehalt eine HBOC-Menge von etwa 5 Gew.-% bis etwa 50 Gew.-% umfasst, wobei der Rest der Zubereitung ein pharmazeutisch annehmbares Verdünnungsmittel umfasst.

3. Flüssige pharmazeutische Formulierung, die ein Oxygenierungskomplement umfasst, umfassend die flüssige HBOC-Zubereitung mit niedrigem Gelöst-Ionengehalt nach Anspruch 1 oder 2 und Blutplasma.

4. Flüssige pharmazeutische Formulierung nach Anspruch 3, wobei das Oxygenierungskomplement eine HBOC-Menge von etwa 5 Gew.-% bis etwa 95 Gew.-% und eine Blutplasmamenge von etwa 5 Gew.-% bis etwa 95 Gew.-% umfasst, wobei die HBOC- und Blutplasmamengen so ausgewählt sind, dass sie zusammen weniger als 100 Gew.-% des Oxygenierungskomplements ausmachen, wobei der Rest, bezogen auf Gewicht, des Oxygenierungskomplements durch ein pharmazeutisch annehmbares Verdünnungsmittel gebildet wird;
wobei beispielsweise das Oxygenierungskomplement eine HBOC-Menge von etwa 5 Gew.-% bis etwa 50 Gew.-% und eine Blutplasmamenge von etwa 5 Gew.-% bis etwa 95 Gew.-% umfasst, wobei die HBOC- und Blutplasmamengen so ausgewählt sind, dass sie zusammen weniger als 100 Gew.-% des Oxygenierungskomplements ausmachen, wobei der Rest, bezogen auf Gewicht, des Oxygenierungskomplements durch ein pharmazeutisch annehmbares Verdünnungsmittel gebildet wird.

5. Flüssige pharmazeutische Formulierung nach Anspruch 3, wobei die pharmazeutische Formulierung Folgendes umfasst:
(i) bis zu etwa 95 Gew.-% des Oxygenierungskomplements, wobei das Oxygenierungskomplement eine HBOC-Menge von etwa 5 Gew.-% bis etwa 95 Gew.-% und eine Blutplasmamenge von etwa 5 Gew.-% bis etwa 95 Gew.-% umfasst, wobei die HBOC- und Blutplasmamengen so gewählt sind, dass sie zusammen weniger als 100 Gew.-% des Oxygenierungskomplements ausmachen, wobei der Rest, bezogen auf Gewicht, des Oxygenierungskomplements durch ein pharmazeutisch annehmbares Verdünnungsmittel gebildet wird; und
wobei der Rest, bezogen auf Gewicht, der pharmazeutischen Formulierung durch einen pharmazeutisch annehmbaren Träger, ein pharmazeutisch annehmbares Verdünnungsmittel oder ein pharmazeutisch annehmbares Hilfsmittel gebildet wird;
beispielsweise (ii) bis zu etwa 95 Gew.-% des Oxygenierungskomplements, wobei das Oxygenierungskomplement eine HBOC-Menge von etwa 5 Gew.-% bis etwa 50 Gew.-% und eine Blutplasmamenge von etwa 5 Gew.-% bis etwa 95 Gew.-% umfasst, wobei die HBOC- und Blutplasmamengen so gewählt sind, dass sie zusammen weniger als 100 Gew.-% des Oxygenierungskomplements ausmachen, wobei der Rest, bezogen auf Gewicht, des Oxygenierungskomplements durch ein pharmazeutisch annehmbares Verdünnungsmittel gebildet wird; und
wobei der Rest, bezogen auf Gewicht, der pharmazeutischen Formulierung durch einen pharmazeutisch annehmbaren Träger, ein pharmazeutisch annehmbares Verdünnungsmittel oder ein pharmazeutisch annehmbares Hilfsmittel gebildet wird.

6. Flüssige pharmazeutische Formulierung nach Anspruch 3, wobei der HBOC polymerisierte Hämoglobinmoleküle mit einer Molekülmasse von mindestens 1.600 kDa umfasst; oder wobei das Hilfsmittel Blutplättchen umfasst.

7. Oxygenierungskomplement, umfassend die flüssige HBOC-Zubereitung mit niedrigem Gelöst-Ionengehalt nach Anspruch 1 oder 2 und Blutplasma, zur Verwendung in einem Verfahren zur Prävention oder Behandlung von hämorrhagischem Schock, wobei das Verfahren das intravenöse Verabreichen des Oxygenierungskomplements, umfassend die flüssige HBOC-Zubereitung mit niedrigem Gelöst-Ionengehalt und Blutplasma, an ein Individuum, das dies benötigt, in einer wirksamen Menge zur Prävention oder Behandlung von hämorrhagischem Schock bei dem Individuum umfasst.

8. Oxygenierungskomplement zur Verwendung in dem Verfahren zur Prävention oder Behandlung von hämorrhagischem Schock nach Anspruch 7, wobei
(i) das Oxygenierungskomplement dem Individuum durch aufeinanderfolgende Verabreichung der flüssigen HBOC-Zubereitung mit niedrigem Gelöst-Ionengehalt und einer flüssigen Blutplasmaformulierung verabreicht wird, wobei die flüssige HBOC-Zubereitung mit niedrigem Gelöst-Ionengehalt im Wesentlichen frei von Blutplasma ist und wobei die flüssige Blutplasmaformulierung im Wesentlichen frei von HBOC ist;
(ii) das Oxygenierungskomplement dem Individuum durch aufeinanderfolgende Verabreichung der flüssigen HBOC-Zubereitung mit niedrigem Gelöst-Ionengehalt und der Blutplasmaformulierung verabreicht wird, wobei die flüssige HBOC-Zubereitung mit niedrigem Gelöst-Ionengehalt vor der Blutplasmaformulierung verabreicht wird;
(iii) das Oxygenierungskomplement dem Individuum durch aufeinanderfolgende Verabreichung der flüssigen HBOC-Zubereitung mit niedrigem Gelöst-Ionengehalt und der Blutplasmaformulierung verabreicht wird, wobei die Blutplasmaformulierung vor der flüssigen HBOC-Zubereitung mit niedrigem Gelöst-Ionengehalt verabreicht wird; oder
(iv) die flüssige HBOC-Zubereitung mit niedrigem Gelöst-Ionengehalt und die Blutplasmaformulierung dem Individuum nicht mehr als etwa 6 Stunden voneinander beabstandet verabreicht werden; oder
(v) das Oxygenierungskomplement verabreicht wird, indem eine flüssige pharmazeutische Formulierung verabreicht wird, die durch Mischen der flüssigen HBOC-Zubereitung mit niedrigem Gelöst-Ionengehalt und des Blutplasmapräparats hergestellt wurde;
gegebenenfalls wobei die flüssige pharmazeutische Formulierung ferner einen pharmazeutisch annehmbaren Träger, ein pharmazeutisch annehmbares Verdünnungsmittel oder ein pharmazeutisch annehmbares Hilfsmittel umfasst.

9. Oxygenierungskomplement zur Verwendung in dem Verfahren zur Prävention oder Behandlung von hämorrhagischem Schock nach Anspruch 8, Teil (v), wobei
(i) das Oxygenierungskomplement eine HBOC-Menge von etwa 5 Gew.-% bis etwa 95 Gew.-% und eine Blutplasmamenge von etwa 5 Gew.-% bis etwa 95 Gew.-% umfasst, wobei die HBOC-und Blutplasmamengen so ausgewählt sind, dass sie zusammen weniger als 100 Gew.-% des Oxygenierungskomplements ausmachen, wobei der Rest, bezogen auf Gewicht, des Oxygenierungskomplements durch ein pharmazeutisch annehmbares Verdünnungsmittel gebildet wird;
(ii) die flüssige pharmazeutische Formulierung bis zu 95 Gew.-% des Oxygenierungskomplements umfasst, wobei das Oxygenierungskomplement eine HBOC-Menge von etwa 5 Gew.-% bis etwa 95 Gew.-% und eine Blutplasmamenge von etwa 5 Gew.-% bis etwa 95 Gew.-% umfasst, wobei die HBOC- und Blutplasmamengen so gewählt sind, dass sie zusammen weniger als 100 Gew.-% des Oxygenierungskomplements ausmachen, wobei der Rest, bezogen auf Gewicht, des Oxygenierungskomplements durch ein pharmazeutisch annehmbares Verdünnungsmittel gebildet wird; und
wobei der Rest, bezogen auf Gewicht, der pharmazeutischen Formulierung durch den pharmazeutisch annehmbaren Träger, das pharmazeutisch annehmbare Verdünnungsmittel oder das pharmazeutisch annehmbare Hilfsmittel gebildet wird;
gegebenenfalls wobei das Hilfsmittel Blutplättchen umfasst;
(iii) das Oxygenierungskomplement eine HBOC-Menge von etwa 5 Gew.-% bis etwa 50 Gew.-% und eine Blutplasmamenge von etwa 5 Gew.-% bis etwa 95 Gew.-% umfasst, wobei die HBOC- und Blutplasmamengen so ausgewählt sind, dass sie zusammen weniger als 100 Gew.-% des Oxygenierungskomplements ausmachen, wobei der Rest, bezogen auf Gewicht, des Oxygenierungskomplements durch ein pharmazeutisch annehmbares Verdünnungsmittel gebildet wird; oder
(iv) die flüssige pharmazeutische Formulierung umfasst bis zu 95 Gew.-% des Oxygenierungskomplements, wobei das Oxygenierungskomplement eine HBOC-Menge von etwa 5 Gew.-% bis etwa 50 Gew.-% und eine Blutplasmamenge von etwa 5 Gew.-% bis etwa 95 Gew.-% umfasst, wobei die HBOC- und Blutplasmamengen so gewählt sind, dass sie zusammen weniger als 100 Gew.-% des Oxygenierungskomplements ausmachen, wobei der Rest, bezogen auf Gewicht, des Oxygenierungskomplements durch ein pharmazeutisch annehmbares Verdünnungsmittel gebildet wird; und
wobei der Rest, bezogen auf Gewicht, der pharmazeutischen Formulierung durch den pharmazeutisch annehmbaren Träger, das pharmazeutisch annehmbare Verdünnungsmittel oder das pharmazeutisch annehmbare Hilfsmittel gebildet wird.

10. Oxygenierungskomplement zur Verwendung in dem Verfahren zur Prävention oder Behandlung von hämorrhagischem Schock nach einem der Ansprüche 7 bis 9, wobei
(i) eine pharmazeutische Formulierung, die Blutplättchen umfasst, zusammen mit dem Oxygenierungskomplement verabreicht wird;
(ii) der hämorrhagische Schock durch einen traumatischen Blutverlust bei dem Individuum verursacht wird; oder
(iii) der hämorrhagische Schock durch einen nichttraumatischen Blutverlust bei dem Individuum verursacht wird.

11. Pharmazeutische Packung, umfassend (i) eine erste Packung, die die flüssige HBOC-Zubereitung mit niedrigem Gelöst-Ionengehalt nach Anspruch 1 oder 2 umfasst, und (ii) eine zweite Packung, die ein Blutplasmapräparat umfasst, und Anweisungen zur Herstellung der flüssigen pharmazeutischen Formulierung nach Anspruch 3.

12. Pharmazeutische Packung nach Anspruch 11, wobei die pharmazeutische Packung ferner (iii) eine dritte Packung, die einen pharmazeutisch annehmbaren Träger, ein pharmazeutisch annehmbares Verdünnungsmittel oder ein pharmazeutisch annehmbares Hilfsmittel umfasst, zum Mischen mit der flüssigen HBOC-Zubereitung mit niedrigem Gelöst-Ionengehalt und dem Blutplasmapräparat umfasst, um dadurch die flüssige pharmazeutische Formulierung zu bilden.

13. Pharmazeutische Packung nach Anspruch 11, wobei das Blutplasmapräparat ein getrocknetes Blutplasmapräparat, aufweisend einen Feuchtigkeitsgehalt von etwa 1 % (w/w) bis etwa 10 % (w/w), ist;
gegebenenfalls wobei die flüssige HBOC-Zubereitung mit niedrigem Gelöst-Ionengehalt nicht mehr als etwa 13,5 mEq/L Natriumionen (Na⁺), nicht mehr als etwa 0,35 mEq/L Kaliumionen (K⁺), nicht mehr als etwa 0,46 mEq/L Calciumionen (Ca²⁺), nicht mehr als etwa 0,15 mEq/L Magnesiumionen (Mg²⁺), nicht mehr als etwa 9,6 mEq/L Chloridionen (Cl-), nicht mehr als 2,4 mEq/L Bicarbonationen (HCO₃²⁻) und nicht mehr als etwa 0,25 mEq/L Hydrogenphosphationen (HPO₄²⁻) umfassen kann.

14. Verfahren zur Herstellung einer flüssigen pharmazeutischen Formulierung nach einem der Ansprüche 3 bis 6, wobei das Verfahren Folgendes umfasst:
Mischen
(i) der flüssigen HBOC-Zubereitung mit niedrigem Gelöst-Ionengehalt nach Anspruch 1 oder 2; und
(ii) eines Blutplasmapräparats;
um dadurch ein Oxygenierungskomplement zu bilden oder eine flüssige pharmazeutische Formulierung zu formulieren.

15. Verfahren zur Herstellung einer flüssigen pharmazeutischen Formulierung nach Anspruch 14, wobei
(a) das Blutplasmapräparat ein getrocknetes Blutplasmapräparat, aufweisend einen Feuchtigkeitsgehalt von etwa 1 % (w/w) bis etwa 10 % (w/w), ist,
gegebenenfalls wobei die flüssige HBOC-Zubereitung mit niedrigem Gelöst-Ionengehalt nicht mehr als etwa 13,5 mEq/L Natriumionen(Na⁺), nicht mehr als etwa 0,35 mEq/L Kaliumionen (K⁺), nicht mehr als etwa 0,46 mEq/L Calciumionen (Ca²⁺), nicht mehr als etwa 0,15 mEq/L Magnesiumionen (Mg2+), nicht mehr als etwa 9,6 mEq/L Chloridionen (Cl-), nicht mehr als 2,4 mEq/L Bicarbonationen (HCO₃²⁻) und nicht mehr als etwa 0,25 mEq/L Hydrogenphosphationen (HPO₄²⁻) umfassen kann;
(b) das Verfahren ferner das Mischen (iii) eines pharmazeutisch annehmbaren Trägers, Verdünnungsmittels oder Hilfsmittels zusammen mit dem Oxygenierungskomplement umfasst, um dadurch eine flüssige pharmazeutische Formulierung zu bilden; oder
(c) die flüssige HBOC-Zubereitung mit niedrigem Gelöst-Ionengehalt und das Blutplasmapräparat 1 Stunde oder weniger vor der Verabreichung an ein Individuum, das dies benötigt, gemischt werden.

## Revendications

1. Préparation de véhicule d'oxygène à base d'hémoglobine (HBOC) liquide à faible teneur en soluté ionique comprenant :
un HBOC,
pas plus d'environ 27 milliéquivalents (mEq/L) d'ions sodium (Na⁺),
pas plus d'environ 0,7 mEq/L d'ions potassium (K⁺),
pas plus d'environ 0,92 mEq/L d'ions calcium (Ca²⁺),
pas plus d'environ 0,3 mEq/L d'ions magnésium (Mg²⁺),
pas plus d'environ 19,2 mEq/L d'ions chlorure (Cl-),
pas plus de 4,8 mEq/L d'ions bicarbonate (HCO₃²⁻), et
pas plus d'environ 0,5 mEq/L d'ions hydrogénophosphate (HPO₄²⁻).

2. Préparation de HBOC liquide à faible teneur en soluté ionique selon la revendication 1, dans laquelle
(i) la préparation de HBOC liquide à faible teneur en soluté ionique ne comprend pas plus d'environ 13,5 mEq/L d'ions sodium (Na⁺), pas plus d'environ 0,35 mEq/L d'ions potassium (K⁺), pas plus d'environ 0,46 mEq/L d'ions calcium (Ca²⁺), pas plus d'environ 0,15 mEq/L d'ions magnésium (Mg²⁺), pas plus d'environ 9,6 mEq/L d'ions chlorure (Cl-), pas plus de 2,4 mEq/L d'ions bicarbonate (HCO₃²⁻) et pas plus d'environ 0,25 mEq/L d'ions hydrogénophosphate (HPO₄²⁻) ;
éventuellement, dans laquelle la préparation de HBOC liquide à faible teneur en soluté ionique ne comprend pas plus d'environ 6,75 mEq/L d'ions sodium (Na⁺), pas plus d'environ 0,175 mEq/L d'ions potassium (K⁺), pas plus d'environ 0,23 mEq/L d'ions calcium (Ca²⁺), pas plus d'environ 0,075 mEq/L d'ions magnésium (Mg²⁺), pas plus d'environ 4,8 mEq/L d'ions chlorure (Cl-), pas plus de 1,2 mEq/L d'ions bicarbonate (HCO₃²⁻) et pas plus d'environ 0,125 mEq/L d'ions hydrogénophosphate (HPO₄²⁻ ) ; et/ou
(ii) la préparation de HBOC liquide à faible teneur en soluté ionique comprend une quantité de HBOC d'environ 5 % à environ 50 % en poids, le reste de la préparation comprenant un diluant pharmaceutiquement acceptable.

3. Formulation pharmaceutique liquide comprenant un complément d'oxygénation comprenant la préparation liquide de HBOC liquide à faible teneur en soluté ionique selon la revendication 1 ou 2, et du plasma sanguin.

4. Formulation pharmaceutique liquide selon la revendication 3, dans laquelle le complément d'oxygénation comprend une quantité de HBOC d'environ 5 % à environ 95 % en poids, et une quantité de plasma sanguin d'environ 5 % à environ 95 % en poids, les quantités de HBOC et de plasma sanguin étant choisies de telle sorte qu'elles constituent ensemble moins de 100 % en poids du complément d'oxygénation, le reste en poids du complément d'oxygénation étant constitué d'un diluant pharmaceutiquement acceptable ;
par exemple, dans laquelle le complément d'oxygénation comprend une quantité de HBOC d'environ 5 % à environ 50 % en poids, et une quantité de plasma sanguin d'environ 5 % à environ 95 % en poids, les quantités de HBOC et de plasma sanguin étant choisies de telle sorte qu'elles constituent ensemble moins de 100 % en poids du complément d'oxygénation, le reste en poids du complément d'oxygénation étant constitué par un diluant pharmaceutiquement acceptable.

5. Formulation pharmaceutique liquide selon la revendication 3, la formulation pharmaceutique comprenant :
(i) jusqu'à environ 95 % en poids du complément d'oxygénation, le complément d'oxygénation comprenant une quantité de HBOC d'environ 5 % à environ 95 % en poids, et une quantité de plasma sanguin d'environ 5 % à environ 95 % en poids, les quantités de HBOC et de plasma sanguin étant choisies de telle sorte qu'elles constituent ensemble moins de 100 % en poids du complément d'oxygénation, le reste en poids du complément d'oxygénation étant constitué par un diluant pharmaceutiquement acceptable ; et
le reste en poids de la formulation pharmaceutique étant constitué par un support, diluant, ou agent auxiliaire pharmaceutiquement acceptable ;
par exemple, (ii) jusqu'à environ 95 % en poids du complément d'oxygénation, le complément d'oxygénation comprenant une quantité de HBOC d'environ 5 % à environ 50 % en poids, et une quantité de plasma sanguin d'environ 5 % à environ 95 % en poids, les quantités de HBOC et de plasma sanguin étant choisies de telle sorte qu'elles constituent ensemble moins de 100 % en poids du complément d'oxygénation, le reste en poids du complément d'oxygénation étant constitué par un diluant pharmaceutiquement acceptable ; et
le reste en poids de la formulation pharmaceutique étant constitué d'un support, diluant ou agent auxiliaire pharmaceutiquement acceptable.

6. Formulation pharmaceutique liquide selon la revendication 3, dans laquelle le HBOC comprend des molécules d'hémoglobine polymérisée ayant une masse moléculaire d'au moins 1 600 kDa ;
ou dans laquelle l'agent auxiliaire comprend des plaquettes sanguines.

7. Complément d'oxygénation comprenant la préparation de HBOC liquide à faible teneur en soluté ionique selon la revendication 1 ou 2 et du plasma sanguin destiné à être utilisé dans un procédé de prévention ou de traitement d'un choc hémorragique, le procédé comprenant l'administration intraveineuse, à un sujet qui en a besoin, du complément d'oxygénation comprenant la préparation de HBOC liquide à faible teneur en soluté ionique et du plasma sanguin, en une quantité efficace pour prévenir ou traiter un choc hémorragique chez le sujet.

8. Complément d'oxygénation destiné à être utilisé dans le procédé de prévention ou de traitement d'un choc hémorragique selon la revendication 7, dans lequel
(i) le complément d'oxygénation est administré au sujet par administration consécutive de la préparation de HBOC liquide à faible teneur en soluté ionique et d'une formulation de plasma sanguin liquide, la préparation de HBOC liquide à faible teneur en soluté ionique étant sensiblement exempte de plasma sanguin, et la formulation de plasma sanguin liquide étant sensiblement exempte de HBOC ;
(ii) le complément d'oxygénation est administré au sujet par administration consécutive de la préparation de HBOC liquide à faible teneur en soluté ionique et de la formulation de plasma sanguin, la préparation de HBOC liquide à faible teneur en soluté ionique étant administrée avant la formulation de plasma sanguin ;
(iii) le complément d'oxygénation est administré au sujet par administration consécutive de la préparation de HBOC liquide à faible teneur en soluté ionique et de la formulation de plasma sanguin, la formulation de plasma sanguin étant administrée avant la préparation de HBOC liquide à faible teneur en soluté ionique ; ou
(iv) la préparation de HBOC liquide à faible teneur en soluté ionique et la formulation de plasma sanguin sont administrées au sujet à pas plus d'environ 6 heures d'écart l'une de l'autre ; ou
(v) le complément d'oxygénation est administré par administration d'une formulation pharmaceutique liquide ayant été préparée par mélange de la préparation de HBOC liquide à faible teneur en soluté ionique et de la préparation de plasma sanguin ;
éventuellement dans lequel la formulation pharmaceutique liquide comprend en outre un support, diluant ou agent auxiliaire pharmaceutiquement acceptable.

9. Complément d'oxygénation destiné à être utilisé dans le procédé de prévention ou de traitement d'un choc hémorragique selon la revendication 8, partie (v), dans lequel
(i) le complément d'oxygénation comprend une quantité de HBOC d'environ 5 % à environ 95 % en poids, et une quantité de plasma sanguin d'environ 5 % à environ 95 % en poids, les quantités de HBOC et de plasma sanguin étant choisies de telle sorte qu'elles constituent ensemble moins de 100 % en poids du complément d'oxygénation, le reste en poids du complément d'oxygénation étant constitué d'un diluant pharmaceutiquement acceptable ;
(ii) la formulation pharmaceutique liquide comprend jusqu'à 95 % en poids du complément d'oxygénation, le complément d'oxygénation comprenant une quantité de HBOC d'environ 5 % à environ 95 % en poids, et une quantité de plasma sanguin d'environ 5 % à environ 95 % en poids, les quantités de HBOC et de plasma sanguin étant choisies de telle sorte qu'elles constituent ensemble moins de 100 % en poids du complément d'oxygénation, le reste en poids du complément d'oxygénation étant constitué par un diluant pharmaceutiquement acceptable ; et
le reste en poids de la formulation pharmaceutique étant constitué par le support, diluant ou agent auxiliaire pharmaceutiquement acceptable ;
éventuellement, dans lequel l'agent auxiliaire comprend des plaquettes sanguines ;
(iii) le complément d'oxygénation comprend une quantité de HBOC d'environ 5 % à environ 50 % en poids, et une quantité de plasma sanguin d'environ 5 % à environ 95 % en poids, les quantités de HBOC et de plasma sanguin étant choisies de telle sorte qu'elles constituent ensemble moins de 100 % en poids du complément d'oxygénation, le reste en poids du complément d'oxygénation étant constitué par un diluant pharmaceutiquement acceptable ; ou
(iv) la formulation pharmaceutique liquide comprend jusqu'à 95 % en poids du complément d'oxygénation, le complément d'oxygénation comprenant une quantité de HBOC d'environ 5 % à environ 50 % en poids, et une quantité de plasma sanguin d'environ 5 % à environ 95 % en poids, les quantités de HBOC et de plasma sanguin étant choisies de telle sorte qu'elles constituent ensemble moins de 100 % en poids du complément d'oxygénation, le reste en poids du complément d'oxygénation étant constitué par un diluant pharmaceutiquement acceptable ; et
le reste en poids de la formulation pharmaceutique étant constitué par le support, diluant ou agent auxiliaire pharmaceutiquement acceptable.

10. Complément d'oxygénation destiné à être utilisé dans le procédé de prévention ou de traitement d'un choc hémorragique selon l'une quelconque des revendications 7 à 9, dans lequel
(i) une formulation pharmaceutique comprenant des plaquettes sanguines est co-administrée avec le complément d'oxygénation ;
(ii) le choc hémorragique est causé par une perte de sang traumatique chez le sujet ; ou
(iii) le choc hémorragique est causé par une perte de sang non traumatique chez le sujet.

11. Emballage pharmaceutique comprenant (i) un premier emballage comprenant la préparation de HBOC liquide à faible teneur en soluté ionique selon la revendication 1 ou 2 et (ii) un second emballage comprenant une préparation de plasma sanguin, et des instructions pour préparer la formulation pharmaceutique liquide selon la revendication 3.

12. Emballage pharmaceutique selon la revendication 11, dans lequel l'emballage pharmaceutique comprend en outre (iii) un troisième emballage comprenant un support, diluant ou agent auxiliaire pharmaceutiquement acceptable pour le mélange avec la préparation de HBOC liquide à faible teneur en soluté ionique et la préparation de plasma sanguin pour former ainsi la formulation pharmaceutique liquide.

13. Emballage pharmaceutique selon la revendication 11, dans lequel la préparation de plasma sanguin est une préparation de plasma sanguin séché ayant une teneur en humidité d'environ 1 % (p/p) à environ 10 % (p/p) ; éventuellement, la préparation de HBOC liquide à faible teneur en soluté ionique ne pouvant pas comprendre plus d'environ 13,5 mEq/L d'ions sodium (Na⁺), pas plus d'environ 0,35 mEq/L d'ions potassium (K⁺), pas plus d'environ 0,46 mEq/L d'ions calcium (Ca²⁺), pas plus d'environ 0,15 mEq/L d'ions magnésium (Mg²⁺), pas plus d'environ 9,6 mEq/L d'ions chlorure (Cl-), pas plus de 2,4 mEq/L d'ions bicarbonate (HCO₃²⁻) et pas plus d'environ 0,25 mEq/L d'ions hydrogénophosphate (HPO₄⁷⁻).

14. Procédé de préparation d'une formulation pharmaceutique liquide selon l'une quelconque des revendications 3 à 6, le procédé comprenant :
le mélange
(i) de la préparation de HBOC liquide à faible teneur en soluté ionique selon la revendication 1 ou 2 ; et
(ii) d'une préparation de plasma sanguin ;
pour constituer ainsi un complément d'oxygénation ou formuler une formulation pharmaceutique liquide.

15. Procédé de préparation d'une formulation pharmaceutique liquide selon la revendication 14, dans lequel
(a) la préparation de plasma sanguin est une préparation de plasma sanguin séché ayant une teneur en humidité d'environ 1 % (p/p) à environ 10 % (p/p),
éventuellement, la préparation de HBOC liquide à faible teneur en soluté ionique ne pouvant pas comprendre plus d'environ 13,5 mEq/L d'ions sodium (Na⁺), pas plus d'environ 0,35 mEq/L d'ions potassium (K⁺), pas plus d'environ 0,46 mEq/L d'ions calcium (Ca²⁺), pas plus d'environ 0,15 mEq/L d'ions magnésium (Mg²⁺), pas plus d'environ 9,6 mEq/L d'ions chlorure (Cl-), pas plus de 2,4 mEq/L d'ions bicarbonate (HCO₃²⁻) et pas plus d'environ 0,25 mEq/L d'ions hydrogénophosphate (HPO₄²⁻) ;
(b) le procédé comprend en outre le mélange de (iii) un support, diluant, ou agent auxiliaire pharmaceutiquement acceptable conjointement avec le complément d'oxygénation pour ainsi former une formulation pharmaceutique liquide ; ou
(c) la préparation de HBOC liquide à faible teneur en soluté ionique et la préparation de plasma sanguin sont mélangées 1 heure ou moins avant l'administration à un sujet en ayant besoin.
